# EUROPÄISCHE PATENTSCHRIFT

(11) **EP 3 135 183 B1**
(45) Veröffentlichungstag und Bekanntmachung des Hinweises auf die Patenterteilung: **06.06.2018**
(21) Anmeldenummer: 16185555.6
(22) Anmeldetag: 24.08.2016
(51) Int. Cl.: A61B 1/00, A61B 17/34

(54) **MIKROINVASIVES MEDIZINISCHES INSTRUMENT**
MICRO-INVASIVE MEDICAL INSTRUMENT
INSTRUMENT MEDICAL MICRO-INVASIF

(30) Priorität: 27.08.2015 DE 102015114264
(43) Veröffentlichungstag der Anmeldung: 01.03.2017
(73) Patentinhaber: Karl Storz SE & Co. KG, 78532 Tuttlingen (DE)
(72) Erfinder: Bärthele, Andreas, 78532 Tuttlingen (DE); Egle, Michael, 78532 Tuttlingen (DE); Wittke, Uwe, 78532 Tuttlingen (DE)

(56) Entgegenhaltungen:
- EP-B1- 1 952 769
- WO-A2-2007/106813
- WO-A2-2014/145595
- DE-A1- 4 211 417
- DE-A1-102008 024 900
- DE-T2- 69 212 259

## Beschreibung

Die vorliegende Erfindung ist auf ein mikroinvasives oder anderes medizinisches Instrument bezogen, insbesondere auf ein Hysteroskop oder ein anderes medizinisches Instrument, bei dem ein erster Bestandteil relativ zu einem zweiten Bestandteil verschiebbar oder auf andere Weise bewegbar und in mindestens einer Position arretierbar ist. Die Erfindung ist insbesondere auf ein derartiges medizinisches Instrument bezogen, bei dem die Arretierung durch einen Arretierungsschieber, der in eine Ausnehmung eingreift, durch manuellen Druck auf eine Drucktaste gelöst werden kann.

Medizinische Instrumente für mikroinvasive oder andere Anwendungen müssen gleichzeitig zahlreiche Anforderungen erfüllen, die einander teils schwer vereinbar sind. Sie müssen robust sein, d.h. auch nach mechanisch grober Behandlung durch medizinisches Personal zuverlässig und präzise wirken. Besonders mikroinvasive medizinische Instrumente bzw. medizinische Instrumente für mikroinvasive Anwendungen müssen ferner in der Regel möglichst kleine Abmessungen und Querschnitte aufweisen. Handhabung und Bedienung sollen einfach, leicht erlernbar und möglichst intuitiv möglich sein. Hinzu kommt eine möglichst weitgehende Zerlegbarkeit auf möglichst einfache und schnelle Weise, um nach einer Verwendung eine möglichst vollständige Reinigung zu ermöglichen.

Unter diesen Bedingungen sind aus anderen Bereichen der Technik bekannte Konstruktionen oft nicht oder allenfalls stark modifiziert verwendbar.

Hierzu schlägt DE 42 11 417 A1 (offenbart den Oberbegriff von Anspruch 1) ein medizinisches Instrument mit einer Wirkeinrichtung vor, die sich in einer Richtung bewegen lässt, sowie beispielsweise einer in einer anderen Richtung bewegbaren Drucktaste.

Mindestens ein Hebel koppelt die Drucktaste und die Wirkeinrichtung, so dass eine Betätigung der Drucktaste eine Bewegung der Wirkeinrichtung in der anderen Richtung bewirkt.

Eine Aufgabe der vorliegenden Erfindung besteht darin, ein verbessertes medizinisches Instrument, insbesondere ein verbessertes mikroinvasives medizinisches Instrument zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, bei einem medizinischen Instrument eine Wippe bzw. einen Hebel zur Übersetzung einer Bewegung in einer ersten Richtung in eine Bewegung in einer entgegengesetzten zweiten Richtung nicht herkömmlich mittels einer Welle oder eines oder mehrerer Achszapfen oder einer Kombination aus Pfanne und Schneide oder eines Festkörpergelenks zu lagern, sondern die resultierende Bewegbarkeit des Hebels in seiner Längsrichtung in Kauf zu nehmen oder auf andere Weise zu unterbinden.

Ein medizinisches Instrument umfasst eine Wirkeinrichtung, die in einer ersten Richtung aus einer ersten Wirkposition in eine zweite Wirkposition bewegbar ist, eine Drucktaste, die manuell in einer zweiten Richtung aus einer ersten Tastenposition in eine zweite Tastenposition bewegbar ist, und einen Hebel, der um eine Schwenkachse schwenkbar ist, wobei die zweite Richtung von der ersten Richtung verschieden ist, wobei der Hebel die Drucktaste und die Wirkeinrichtung derart mechanisch koppelt, dass eine Bewegung der Drucktaste in der zweiten Richtung mit einer Bewegung der Wirkeinrichtung in der ersten Richtung einher geht, und wobei der Hebel im Bereich der Schwenkachse keine zu seiner Schwenkachse rotationssymmetrische Fläche aufweist.

Das medizinische Instrument ist insbesondere ein mikroinvasives medizinisches Instrument bzw. ein medizinisches Instrument zur Verwendung im Rahmen von mikroinvasiven Maßnahmen. Die Wirkeinrichtung ist insbesondere derart geführt, dass sie nur oder im Wesentlichen (d. h. von Spiel abgesehen) nur in der ersten Richtung oder entgegengesetzt dazu bewegt werden kann. Die Drucktaste ist insbesondere derart geführt, dass sie nur oder im Wesentlichen (d. h. von Spiel abgesehen) nur in der zweiten Richtung oder entgegengesetzt dazu bewegt werden kann. Die Schwenkachse, um die der Hebel schwenkbar ist, ist insbesondere orthogonal zur ersten Richtung und zur zweiten Richtung. Die zweite Richtung ist insbesondere zur ersten Richtung entgegengesetzt.

Der Hebel ist im Bereich der Schwenkachse insbesondere schlicht balken- oder stabförmig bzw. weist eine zylindrische Gestalt mit einem kreisförmigen, rechteckigen oder anderen Querschnitt und einer Zylinderachse orthogonal zur Schwenkachse auf. Insbesondere ist der gesamte Hebel zylindrisch oder im Wesentlichen zylindrisch mit einer Symmetrieachse orthogonal zur Schwenkachse des Hebels.

Der Hebel weist insbesondere keine Welle, keinen Achszapfen und keine Bohrung zur Aufnahme einer Welle auf. Der Hebel weist ferner insbesondere keine Pfanne und keine Schneide zur Lagerung auf.

Durch den Verzicht auf eine Welle, einen Achszapfen, eine Pfanne, eine Schneide oder eine Bohrung an dem Hebel zur Definition der Schwenkachse können die Fertigung des Hebels deutlich vereinfacht und eine weitergehende Miniaturisierung ermöglicht werden. Ferner kann durch den Verzicht auf eine Welle, eine Bohrung, einen Achszapfen, eine Pfanne und eine Schneide das Zusammensetzen des medizinischen Instruments vereinfacht werden. Insbesondere kann der Hebel so ausgebildet sein, dass eine bestimmte Orientierung des Hebels (im Sinne einer Rotation um seine Längsachse) beim Einsetzen in das medizinische Instrument nicht erforderlich ist.

Die Verschiebbarkeit des Hebels, die aus dem Fehlen eines herkömmlichen Lagers resultiert, kann insbesondere bei einer Auslegung des medizinischen Instruments derart, dass der Hebel bei der vorgesehenen Verwendung nur um kleine Winkel geschwenkt wird, vernachlässigt werden. Alternativ und insbesondere bei Auslegung des medizinischen Instruments derart, dass ein Schwenken des Hebels um nicht nur kleine Winkel vorgesehen ist, können im Folgenden beschriebene Maßnahmen die vorgesehen Anordnung des Hebels gewährleisten.

Bei einem medizinischen Instrument, wie es hier beschrieben ist, ragt ein mittlerer Bereich des Hebels insbesondere durch eine Öffnung in einer Wand zwischen der Drucktaste und der Wirkeinrichtung hindurch, wobei der mittlere Bereich des Hebels durch die Öffnung in der Wand mechanisch so geführt ist, dass der Hebel um die Schwenkachse schwenkbar, jedoch nicht oder nicht wesentlich in der ersten Richtung oder der zweiten Richtung verschiebbar ist.

Der Hebel ist nicht wesentlich in der ersten Richtung verschiebbar, wenn die Distanz, über die der Hebel parallel zur ersten Richtung verschiebbar ist, klein ist im Verhältnis zu den Wegen, die die Enden des Hebels zwischen den vorgesehenen extremen Winkelpositionen des Hebels zurücklegen, also beispielsweise nur die Hälfte, ein Drittel, ein Fünftel oder ein Zehntel beträgt. Der Hebel und die Öffnung sind insbesondere derart ausgebildet, dass in einer oder in beiden vorgesehenen extremen Winkelpositionen des Hebels diese gleichzeitig an zwei in der ersten Richtung und/oder in der zweiten Richtung einander gegenüberliegenden Bereichen des Rands der Öffnung anliegt. Dadurch kann das von dem Hebel vermittelte Übersetzungsverhältnis zwischen Drucktaste und Wirkeinrichtung zumindest bezogen auf den gesamten vorgesehenen Schwenkbereich des Hebels exakt definiert sein.

Bei einem medizinischen Instrument, wie es hier beschrieben ist, weist die Wand in der Umgebung der Öffnung insbesondere eine reduzierte Dicke auf.

Die Dicke der Wand kann in der Umgebung der Öffnung beispielsweise durch eine konische Bohrung oder durch eine zylindrische Bohrung, welche die Wand nicht oder nur im Bereich der Öffnung vollständig durchdringt, reduziert sein. Die Dicke der Wand ist in der Umgebung der Öffnung beispielsweise auf höchstens die Hälfte, höchstens ein Drittel, höchstens ein Fünftel oder höchstens ein Zehntel reduziert.

Durch eine im Bereich der Öffnung reduzierte Dicke der Wand können ein größerer Schwenkbereich des Hebels und/oder eine verbesserte Führung des Hebels auch zwischen seinen extremen Winkelpositionen erzielt werden.

Bei einem medizinischen Instrument, wie es hier beschrieben ist, weist der Hebel insbesondere ein erstes Ende, das in eine Ausnehmung in der Wirkeinrichtung eingreift, und ein zweites Ende, das in eine Ausnehmung in der Drucktaste eingreift, auf.

Das erste Ende des Hebels und die Ausnehmung in der Wirkeinrichtung sind insbesondere derart ausgebildet, dass das erste Ende des Hebels in der Ausnehmung in der Wirkeinrichtung reibungs- und vor allem spielarm geführt ist. Das zweite Ende des Hebels und die Ausnehmung in der Drucktaste sind insbesondere derart ausgebildet, dass das zweite Ende des Hebels in der Ausnehmung in der Drucktaste reibungs- und vor allem spielarm geführt ist.

Die Ausnehmung in der Wirkeinrichtung ist beispielsweise durch eine Sackbohrung oder eine einseitig verschlossene Durchgangsbohrung gebildet. Die Ausnehmung in der Drucktaste ist beispielsweise durch eine Sackbohrung oder eine einseitig verschlossene Durchgangsbohrung gebildet. Insbesondere ist zumindest eine der beiden Ausnehmungen durch eine Durchgangsbohrung gebildet, die nach dem Einsetzen des Hebels einseitig verschlossen wurde.

Wenn sowohl die Ausnehmung in der Wirkeinrichtung als auch die Ausnehmung in der Drucktaste jeweils nur eine Öffnung aufweisen, also beispielsweise nicht die Gestalt von Durchgangsbohrungen, sondern von Sackbohrungen aufweisen, können die Ausnehmung in der Wirkeinrichtung und die Ausnehmung in der Drucktaste so ausgebildet sein, dass der Hebel jederzeit im Wesentlichen in seiner vorgesehenen Position gehalten wird. Insbesondere kann der Hebel in diesem Fall nicht oder nicht allein durch eine Bewegung in Richtung parallel zu seiner Längsrichtung oder Längsachse von seiner vorgesehenen Position entfernt werden.

Ein medizinisches Instrument, wie es hier beschrieben ist, umfasst insbesondere ferner einen O-Ring aus einem elastischen Material, wobei entweder der O-Ring am zweiten Ende des Hebels anliegt und das erste Ende des Hebels in die Ausnehmung in der Wirkeinrichtung schiebt oder der O-Ring am ersten Ende des Hebels anliegt und das zweite Ende des Hebels in die Ausnehmung in der Drucktaste schiebt.

Der O-Ring ist insbesondere aus Gummi bzw. Naturkautschuk oder synthetischem Kautschuk oder einem anderen Elastomer oder einem anderen elastischen Material gebildet.

Indem der O-Ring an einem Ende des Hebels anliegt und der Hebel in seiner Längsrichtung oder im Wesentlichen in seiner Längsrichtung zum gegenüberliegenden Ende hin schiebt, kann er eine definierte Position des Hebels gewährleisten. Ein O-Ring kann auch bei hoher Qualität ein kostengünstiges Bauteil sein und die Fertigung des medizinischen Instruments deutlich vereinfachen, insbesondere im Vergleich zu einer Lagerung des Hebels mittels einer Welle, eines oder mehrerer Achszapfen oder einer Kombination aus Pfanne und Schneide.

Bei einem medizinischen Instrument, wie es hier beschrieben ist, ist der Hebel insbesondere so ausgebildet, dass innerhalb seines gesamten vorgesehenen Schwenkbereichs beide Enden des Hebels gleichzeitig an einander gegenüberliegenden Flächen anliegen.

Die einander gegenüberliegenden und insbesondere parallelen oder im Wesentlichen parallelen Flächen, an denen die Enden des Hebels innerhalb seines gesamten Schwenkbereichs oder eines großen Teils seines Schwenkbereichs gleichzeitig anliegen oder im Wesentlichen anliegen, sind insbesondere Teilbereiche der inneren Oberflächen der erwähnten Ausnehmungen in der Wirkeinrichtung und in der Drucktaste.

Um ein gleichzeitiges Anliegen beider Enden des Hebels zu ermöglichen, sind die Enden des Hebels insbesondere als Ausschnitte der Oberfläche eines Kreiszylinders oder als Ausschnitte von Oberflächen von Kreiszylindern, deren Achsen zusammenfallen, oder als Ausschnitte der Oberfläche einer Kugel oder als Ausschnitte von Oberflächen von Kugeln, deren Mittelpunkte zusammenfallen, ausgebildet. Eine Ausgestaltung der Enden des Hebels als Ausschnitte der Oberfläche einer Kugel oder von Oberflächen zweier Kugeln mit identischem Mittelpunkt kann eine einwandfreie Funktion des Hebels auch bei beliebiger Rotation des Hebels um seine Längsachse ermöglichen. Die Achse oder die zusammenfallenden Achsen der Kreiszylinder oder der Mittelpunkt oder die zusammenfallenden Mittelpunkte der Kugeln können in der Ebene der erwähnten Öffnung in einer Wand zwischen der Drucktaste und der Wirkeinrichtung liegen. Alternativ können die zusammenfallenden Achsen der Kreiszylinder oder die zusammenfallenden Mittelpunkte der Kugeln außerhalb der erwähnten Öffnung in der Wand zwischen Drucktaste und Wirkeinrichtung angeordnet sein.

Bei einem medizinischen Instrument, wie es hier beschrieben ist, ist die Wirkeinrichtung insbesondere ein Arretierungsschieber oder mit einem Arretierungsschieber mechanisch gekoppelt, wobei der Arretierungsschieber zum formschlüssigen Halten eines Bestandteils des medizinischen Instruments in einer vorbestimmten Position vorgesehen ist.

Der Arretierungsschieber kann klinkenförmig bzw. mit einer geneigten Flanke ausgebildet sein, so dass er den Bestandteil des medizinischen Instruments nur gegen Kräfte in einer Richtung, nicht jedoch bei einer ausreichend großen Kraft in der entgegengesetzten Richtung in der vorbestimmten Position hält. Alternativ kann der Arretierungsschieber ausgebildet sein, um den Bestandteil des medizinischen Instruments in zwei entgegengesetzten Richtung in der vorbestimmten Position zu halten. Der Arretierungsschieber kann ferner zum alternativen formschlüssigen Halten des Bestandteils des medizinischen Instruments in einer von mehreren vorbestimmten Positionen vorgesehen sein. Der Arretierungsschieber ist insbesondere zum Halten des Bestandteils in einer oder mehreren vorbestimmten Positionen durch formschlüssigen Eingriff in eine Ausnehmung vorgesehen.

Bei einem medizinischen Instrument, wie es hier beschrieben ist, umgibt die Wand insbesondere die Wirkeinrichtung ringförmig, wobei ein Bereich der Drucktaste die Wand ringförmig umgibt.

Die Wand weist insbesondere im Wesentlichen (d.h. beispielsweise abgesehen von der erwähnten Öffnung, durch die der Hebel hindurch ragt) die Gestalt eines Teils einer Mantelfläche eines Kreiszylinders oder eines anderen Zylinders auf. Die Drucktaste oder ein Bauteil der Drucktaste weist insbesondere eine becherförmige Gestalt auf, wobei der Rand der Drucktaste die Wand ringförmig umgibt.

Bei einem medizinischen Instrument, wie es hier beschrieben ist, sind insbesondere die Wirkeinrichtung, die Wand und die Drucktaste abschnittsweise koaxial angeordnet.

Eine koaxiale Anordnung kann eine besonders kompakte Bauweise ermöglichen.

Bei einem medizinischen Instrument, wie es hier beschrieben ist, ist die Wirkeinrichtung insbesondere formschlüssig durch die Wand geführt, wobei die Drucktaste formschlüssig durch die Wand geführt ist.

Die Wirkeinrichtung ist durch die Wand insbesondere derart spiel- und reibungsarm geführt, dass eine Bewegung der Wirkeinrichtung im Wesentlichen nur in der ersten Richtung und in dazu entgegengesetzter Richtung möglich ist. Optional kann die Wirkeinrichtung zusätzlich um die erste Richtung rotierbar sein. Die Rotation der Wirkeinrichtung um die erste Richtung kann jedoch insbesondere durch den Hebel unterbunden oder auf einen kleinen Winkelbereich beschränkt sein.

Die Drucktaste ist durch die Wand insbesondere derart spiel- und reibungsarm geführt, dass eine Bewegung der Drucktaste im Wesentlichen nur in der zweiten Richtung und in dazu entgegengesetzter Richtung möglich ist. Optional kann die Drucktaste ferner um die zweite Richtung rotierbar sein. Diese Rotation der Drucktaste um die zweite Richtung ist jedoch insbesondere durch den Hebel unterbunden oder auf einen kleinen Winkelbereich beschränkt.

Eine formschlüssige Führung sowohl der Wirkeinrichtung als auch der Drucktaste durch die Wand ist insbesondere in einer (teilweisen) koaxialen Anordnung möglich. Die formschlüssige Führung sowohl der Wirkeinrichtung als auch der Drucktaste durch die Wand kann eine besonders kompakte Ausgestaltung ermöglichen.

Bei einem medizinischen Instrument, wie es hier beschrieben ist, umfasst die Drucktaste insbesondere ein becherförmiges Bauteil und ein in einem Hohlraum des becherförmigen Bauteils eingesetztes ringförmiges Bauteil, wobei die Ausnehmung in der Drucktaste in dem ringförmigen Bauteil ausgebildet ist.

Das becherförmige Bauteil weist einen Rand auf, der die offene Seite des becherförmigen Bauteils ringförmig umschließt und insbesondere dem unten erwähnten Außenschaft zugewandt ist. Das ringförmige Bauteil ist insbesondere derart in das becherförmige Bauteil eingesetzt, dass ein Rand des ringförmigen Bauteils mit dem Rand des becherförmigen Bauteils bündig oder im Wesentlichen bündig angeordnet ist. Das ringförmige Bauteil ist insbesondere in das becherförmige Bauteil geschraubt und/oder durch eine umlaufende Schweiß- oder Klebenaht mit ihm verbunden.

Die Ausnehmung in der Drucktaste ist insbesondere durch eine sich in radialer Richtung erstreckende Durchgangsbohrung im ringförmigen Bauteil gebildet, wobei das radial äußere Ende der Durchgangsbohrung durch die Wand des becherförmigen Bauteils verschlossen ist.

Bei einem medizinischen Instrument, wie es hier beschrieben ist, weist das ringförmige Bauteil insbesondere eine ringförmig an seinem äußeren Umfang umlaufende Nut auf, in die der O-Ring eingelegt ist.

Die Nut schneidet insbesondere die Ausnehmung in der Drucktaste.

Bei einem medizinischen Instrument, wie es hier beschrieben ist, ist die Ausnehmung in der Drucktaste insbesondere durch eine fensterförmige Öffnung oder eine Durchgangsbohrung in dem ringförmigen Bauteil gebildet.

Wenn die Ausnehmung in der Drucktaste durch eine Durchgangsbohrung in dem ringförmigen Bauteil gebildet ist, liegt die Achse der Durchgangsbohrung insbesondere teilweise innerhalb der Nut. Beispielsweise liegt der Flächenschwerpunkt eines Querschnitts der Nut auf der Achse der Durchgangsbohrung im ringförmigen Bauteil.

Das becherförmige Bauteil kann nach dem Einsetzen des Hebels in die Durchgangsbohrung im ringförmigen Bauteil, die Öffnung in der Wand und die Ausnehmung in der Wirkeinrichtung und dem nachfolgenden Einlegen des O-Rings in die außen umlaufende Nut sowohl die Nut als auch die Durchgangsbohrung im ringförmigen Bauteil verschließen. Dadurch kann das becherförmige Bauteil den O-Ring vor schädlichen mechanischen und anderen Einwirkungen schützen und - zusammen mit dem O-Ring - den Hebel an der vorgesehenen Position halten.

Bei einem medizinischen Instrument, wie es hier beschrieben ist, weist die Durchgangsbohrung insbesondere einen zur Außenseite des ringförmigen Bauteils hin stufenförmig oder kontinuierlich zunehmenden Querschnitt auf.

Insbesondere ist die Durchgangsbohrung zumindest abschnittsweise konisch. Ein zur Außenseite des ringförmigen Bauteils hin zunehmender Querschnitt kann eine insbesondere in der zweiten Richtung spielärmere Führung des radial äußeren Endes des Hebels und/oder einen größeren Schwenkbereich des Hebels ermöglichen.

Bei einem medizinischen Instrument, wie es hier beschrieben ist, unterbinden insbesondere Formschluss zwischen dem Hebel und der Drucktaste und Formschluss zwischen dem Hebel und der Öffnung in der Wand ein Abziehen der Drucktaste von dem medizinischen Instrument.

Der Hebel kann somit gleichzeitig zwei Funktionen aufweisen, nämlich die Übersetzung der Bewegung der Drucktaste in die (insbesondere entgegengesetzte) Bewegung der Wirkeinrichtung und das Halten der Drucktaste am medizinischen Instrument. Dadurch können eine weitere Einrichtung zum Halten der Drucktaste am medizinischen Instrument und Bauraum eingespart und die Fertigung des medizinischen Instruments vereinfacht werden.

Ein medizinisches Instrument, wie es hier beschrieben ist, umfasst insbesondere ferner eine Feder oder eine andere elastische Einrichtung zwischen der Wirkeinrichtung oder dem Außenschaft oder einem die Wand bildenden rohrförmigen Bereich am Außenschaft einerseits und der Drucktaste andererseits.

Die Feder oder andere elastische Einrichtung kann die Drucktaste in die erste Tastenposition schieben, von der aus die Drucktaste manuell gegen die Kraft der Feder oder der anderen elastischen Einrichtung zu der zweiten Tastenposition bewegt werden kann. Die Feder oder die andere elastische Einrichtung kann mittelbar über den Hebel oder durch unmittelbare Einwirkung auf die Wirkeinrichtung die Wirkeinrichtung in die erste Wirkposition schieben. Die Wirkeinrichtung kann in der ersten Wirkposition beispielsweise in eine Ausnehmung eingreifen, um einen Bestandteil des medizinischen Instruments in einer vorbestimmten Position relativ zu einem anderen Bestandteil des medizinischen Instruments zu halten.

Ein medizinisches Instrument, wie es hier beschrieben ist, umfasst insbesondere ferner eine Einrichtung zum Unterbinden einer Rotation der Drucktaste relativ zu dem rohrförmigen Bereich am Außenschaft oder relativ zu der Wirkeinrichtung.

Die Einrichtung zum Unterbinden einer Rotation umfasst beispielsweise einen Stift oder einen Steg an der Drucktaste, der bzw. die in eine korrespondierende Nut parallel zur zweiten Richtung an der Wand eingreift. Alternativ umfasst die Einrichtung zum Unterbinden einer Rotation beispielsweise einen Stift oder einen Steg an der Wand oder einem anderen Bereich des medizinischen Instruments, die in eine Nut parallel zur zweiten Richtung in der Drucktaste eingreift. Im Fall des beschriebenen Aufbaus der Drucktaste aus einem becherförmigen Bauteil und einem ringförmigen Bauteil ist die Einrichtung zum Unterbinden einer Rotation insbesondere teilweise am ringförmigen Bauteil vorgesehen.

Ein medizinisches Instrument, wie es hier beschrieben ist, ist insbesondere ein Hysteroskop mit einem Endoskop und einem Außenschaft, wobei das Endoskop relativ zum Außenschaft in axialer Richtung verschiebbar ist, wobei die Wirkeinrichtung eine Arretiereinrichtung ist, und wobei die Wirkeinrichtung vorgesehen und ausgebildet ist, um in der ersten Wirkposition in eine Ausnehmung am Endoskop einzugreifen, um so das Endoskop in einer vorbestimmten Position relativ zum Außenschaft zu arretieren.

Die axiale Richtung ist die Richtung parallel zu den Längsachsen des Endoskops und des Außenschafts. Die Wirkeinrichtung ist insbesondere nahe dem proximalen Ende des Außenschafts angeordnet. Die Wirkeinrichtung ist insbesondere relativ zum Außenschaft ausschließlich in der ersten Richtung, die insbesondere orthogonal zur axialen Richtung bzw. zu den Längsachsen von Endoskop und Außenschaft ist, und in entgegengesetzter Richtung bewegbar geführt.

Ein Verfahren zum Zusammensetzen eines medizinischen Instruments umfasst Schritte des Einsetzens einer Wirkeinrichtung in einen rohrförmigen Bereich des medizinischen Instruments, des Einführens des rohrförmigen Bereichs in ein ringförmiges Bauteil, des Einführens eines Hebels in eine Ausnehmung in dem ringförmigen Bauteil, in eine Öffnung im rohrförmigen Bereich und in eine Ausnehmung in der Wirkeinrichtung und des Einsetzens des ringförmigen Bauteils in ein becherförmiges Bauteil.

Der rohrförmige Bereich des medizinischen Instruments ist insbesondere an einem Außenschaft des medizinischen Instruments vorgesehen und orthogonal oder im Wesentlichen orthogonal zur Längsachse des Außenschafts angeordnet. Das Einsetzen des ringförmigen Bauteils in das becherförmige Bauteil entspricht einem Überstülpen des becherförmigen Bauteils über das ringförmige Bauteil. Das Einsetzen des ringförmigen Bauteils in das becherförmige Bauteil umfasst insbesondere ein Einschrauben des ringförmigen Bauteils in das becherförmige Bauteil bzw. ein Aufschrauben des becherförmigen Bauteils auf das ringförmige Bauteil.

Das Verfahren ist zum Zusammensetzen eines medizinischen Instruments, wie es hier beschrieben ist, vorgesehen und ausgebildet.

Das Verfahren umfasst insbesondere ferner zwischen dem Schritt des Einführens des rohrförmigen Bereichs in das ringförmige Bauteil und dem Schritt des Einführens des Hebels einen Schritt des Ausrichtens der Wirkeinrichtung und des ringförmigen Bauteils relativ zu dem rohrförmigen Bereich derart, dass die Ausnehmung in dem ringförmigen Bauteil, die Öffnung im rohrförmigen Bereich und die Ausnehmung im Arretierungsschieber fluchten.

Das Verfahren umfasst insbesondere ferner zwischen den Schritten des Einführens des Hebels und des Einsetzens des ringförmigen Bauteils in das becherförmige Bauteil einen Schritt des Einlegens eines O-Rings in eine ringförmig am äußeren Umfang des ringförmigen Bauteils umlaufende Nut.

Das beschriebene Verfahren und seine Varianten ermöglichen mit vergleichsweise wenigen Schritten und aus vergleichsweise wenigen Bauteilen eine Fertigung beispielsweise einer Einrichtung zum lösbaren Arretieren eines Außenschafts in einer oder mehreren vorbestimmten, alternativen Position relativ zu einem Endoskop.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine weitere schematische Darstellung des medizinischen Instruments aus Figur 1;
- Figur 3: eine schematische Schnittdarstellung einer Arretiereinrichtung;
- Figur 4: eine weitere schematische Schnittdarstellung der Arretiereinrichtung aus Figur 3;
- Figur 5: eine schematische Schnittdarstellung von Bestandteilen der Arretiereinrichtung aus den Figuren 3 und 4;
- Figur 6: eine weitere schematische Schnittdarstellung von Bestandteilen der Arretiereinrichtung aus den Figuren 3 bis 5;
- Figur 7: eine weitere schematische Schnittdarstellung der Arretiereinrichtung aus den Figuren 3 bis 6;
- Figur 8: eine schematische Schnittdarstellung einer weiteren Arretiereinrichtung;
- Figur 9: eine schematische Schnittdarstellung einer weiteren Arretiereinrichtung;
- Figur 10: eine weitere schematische Schnittdarstellung der Arretiereinrichtung aus Figur 9;
- Figur 11: eine schematische Schnittdarstellung einer weiteren Arretiereinrichtung;
- Figur 12: eine weitere schematische Schnittdarstellung der Arretiereinrichtung aus Figur 11;
- Figur 13: ein schematisches Flussdiagramm eines Verfahrens zum Zusammensetzen eines medizinischen Instruments.

### Beschreibung der Ausführungsformen

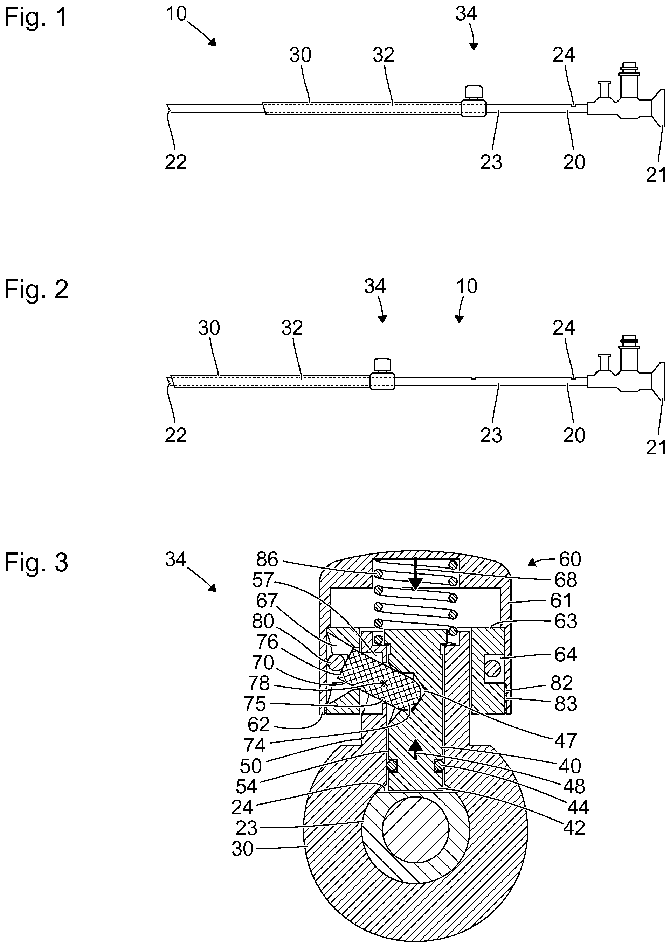

Figur 1 zeigt eine schematische Darstellung eines medizinischen Instruments 10. Das medizinische Instrument 10 weist zwei relativ zueinander bewegbare Teile auf. Beim dargestellten Beispiel sind dies ein Endoskop 20 oder eine ähnliche Vorrichtung, die Merkmale, Eigenschaften und Funktionen eines Endoskops aufweisen kann, und ein Außenschaft 30. Das Endoskop 20 weist ein proximales Ende 21 - beispielsweise gebildet durch ein Okular - und ein distales Ende sowie einen langen dünnen Schaft 23 auf. Der Schaft 23 des Endoskops 20 weist eine oder mehrere Ausnehmungen 24 auf, von denen in Figur 1 nur eine sichtbar ist.

Der Außenschaft 30 weist einen im Wesentlichen zylindrischen Hohlraum 32 auf, dessen Konturen in Figur 1 durch gestrichelte Linien angedeutet sind. Der Querschnitt des zylindrischen Hohlraums 32 des Außenschafts 30 und der Querschnitt des Schafts 23 des Endoskops 20 sind insbesondere so ausgebildet, dass der Schaft 23 des Endoskops 20 in Richtung parallel zu seiner Längsachse im Außenschaft 30 verschiebbar und spiel- und reibungsarm geführt ist.

Der Außenschaft 30 weist ferner eine Arretiereinrichtung 34 auf, die bei dem dargestellten Beispiel am proximalen Ende des Außenschafts 30 angeordnet ist. Die Arretiereinrichtung 34 weist einen in Figur 1 nicht sichtbaren Arretierungsschieber auf, der zur Arretierung des Außenschafts 30 in einer oder mehreren vorbestimmten Positionen am Endoskop 20 in eine Ausnehmung 24 oder - wie nachfolgend beispielhaft angenommen - alternativ in eine von mehreren Ausnehmungen 24 am Schaft 23 des Endoskops 20 eingreifen kann.

Das medizinische Instrument 10 ist insbesondere ein Hysteroskop zur optischen Untersuchung der Gebärmutter. Der Außenschaft 30 kann während des Einführens des Hysteroskops und in verschiedenen Phasen der Untersuchung in verschiedenen Positionen arretiert werden, die durch die Ausnehmungen 24 definiert sind.

Figur 2 zeigt eine weitere schematische Darstellung des medizinischen Instruments 10 aus Figur 1. Die Art der Darstellung entspricht derjenigen der Figur 1.

In Figur 2 ist der Außenschaft 30 im Vergleich zu der in Figur 1 gezeigten Situation in einer nach distal verschobenen Position gezeigt, bei der der Arretierungsschieber der Arretiereinrichtung 34 in eine nicht sichtbare Ausnehmung im Schaft 23 des Endoskops 20 eingreift. In Figur 2 ist die Ausnehmung 24 im Schaft 23 des Endoskops 20 sichtbar, in die der Arretierungsschieber in der in Figur 1 gezeigten Position des Außenschafts 30 eingreifen kann.

Figur 3 zeigt eine schematische Darstellung eines Schnitts durch eine Arretiereinrichtung 34, beispielsweise durch die Arretiereinrichtung des anhand der Figuren 1 und 2 dargestellten medizinischen Instruments. Die Schnittebene der Figur 3 ist orthogonal zu den Zeichenebenen der Figuren 1 und 2 und orthogonal zur Längsachse des Schafts 23 des Endoskops 20. Die Schnittebene der Figur enthält eine Symmetrieachse zahlreicher Merkmale der Arretiereinrichtung 34.

Wie bereits erwähnt umfasst die Arretiereinrichtung 34 als Wirkeinrichtung einen Arretierungsschieber 40 mit einem Wirkende 42. Bei der in Figur 3 dargestellten Wirkposition des Arretierungsschiebers 40 greift das Wirkende 42 des Arretierungsschiebers 40 in die Ausnehmung 24 im Schaft 23 des Endoskops ein. Dadurch ist eine vorbestimmte Position des Außenschafts 30 relativ zum Endoskops 20 (vgl. Figuren 1, 2) definiert.

Der Arretierungsschieber 40 weist ferner einen O-Ring 44 in einer nahe dem Wirkende 42 umlaufenden Nut auf. Der O-Ring 44 kann ein Eindringen von verunreinigenden und die Funktionsfähigkeit der Arretiereinrichtung 34 gefährdenden Substanzen in die Arretiereinrichtung 34 unterbinden oder erschweren.

Der Arretierungsschieber 40 weist ferner eine Ausnehmung 47 auf. Die Ausnehmung 47 im Arretierungsschieber 40 ist insbesondere durch eine Bohrung in einer Richtung orthogonal zur Längsachse des Arretierungsschiebers 40 gebildet. Die die Ausnehmung 47 bildende Bohrung weist in einem äußeren Bereich eine konische Gestalt und in der Mitte des Arretierungsschiebers 40 bzw. nahe der Symmetrieachse des Arretierungsschiebers 40 einen zylindrischen Abschnitt auf.

Der Arretierungsschieber 40 ist in einem im Wesentlichen rohrförmigen Bereich 50 am Außenschaft 30 angeordnet. Der rohrförmige Bereich 50 ist insbesondere an einem Ende des Außenschafts 30 mit vergrößerter Wandstärke angeordnet. Der rohrförmige Bereich 50 weist im Wesentlichen die Gestalt einer Mantelfläche eines Kreiszylinders auf und umschließt eine Bohrung bzw. einen Hohlraum 54, in dem der Arretierungsschieber 40 angeordnet ist. Der äußere Querschnitt des Arretierungsschiebers 40 und der Querschnitt des Hohlraums 54 im rohrförmigen Bereich 50 sind so ausgebildet, dass der Arretierungsschieber 40 im Hohlraum 54 spiel- und reibungsarm geführt ist.

Durch eine Bewegung des Arretierungsschiebers 40 in einer ersten Richtung 48 kann das Wirkende 42 des Arretierungsschiebers 40 aus der Ausnehmung 24 im Schaft 23 des Endoskops herausgehoben werden, um die Arretierung des Außenschafts 30 relativ zum Schaft 23 des Endoskops aufzuheben. Die Richtung 48 ist orthogonal oder im Wesentlichen orthogonal zur Längsachse des Schafts 23 des Endoskops.

Abweichend von einer rein rohrförmigen Gestalt weist der rohrförmige Bereich 50 eine Öffnung 57 auf. Die Öffnung 57 ist insbesondere erzeugt durch eine Bohrung in einer Richtung orthogonal zur ersten Richtung 48. Die Bohrung weist eine zumindest abschnittsweise eine konische Gestalt und/oder zumindest abschnittsweise einen rechteckigen Längsschnitt auf. Der Querschnitt der Bohrung nimmt von außen (in Figur 3: links) nach innen (in Figur 3: rechts) kontinuierlich oder stufenweise ab.

Die Arretiereinrichtung 34 umfasst ferner eine Drucktaste 60 mit einem becherförmigen Bauteil 61. Das becherförmige Bauteil 61 weist einen Rand 62, der dem Außenschaft 30 zugewandt ist, auf. Im becherförmigen Bauteil 61 ist ein ringförmiges Bauteil 63 angeordnet, das bei dem dargestellten Beispiel mit dem Rand 62 des becherförmigen Bauteils 61 bündig oder im Wesentlichen bündig abschließt.

An der Innenseite des becherförmigen Bauteils 61 nahe dessen Rand 62 ist ein Innengewinde 82 vorgesehen. Am äußeren Umfang des ringförmigen Bauteils 63 ist ein zum Innengewinde 82 korrespondierendes Außengewinde 83 vorgesehen. Das ringförmige Bauteil 63 ist mit dem becherförmigen Bauteil 61 durch eine Schraubverbindung zwischen dem Außengewinde 83 und dem Innengewinde 82 mechanisch verbunden. Alternativ oder zusätzlich kann das ringförmige Bauteil 63 durch eine umlaufende Schweißnaht, durch Schweißpunkte, durch eine Klebung oder durch Pressung oder auf andere Weise form-, stoff- und/oder kraftschlüssig verbunden sein.

Das ringförmige Bauteil 63 weist an seinem äußeren Umfang eine ringförmig umlaufende Nut 64 auf. In der ringförmigen Nut 64 liegt ein O-Ring 80 aus einem elastischen Material. Ferner weist das ringförmige Bauteil 63 eine Ausnehmung 67 auf. Die Ausnehmung 67 ist insbesondere durch eine Bohrung in einer Richtung orthogonal zur ersten Richtung 48, in der der Arretierungsschieber 40 bewegbar ist, gebildet. Die Ausnehmung 67 weist einen abschnittsweise konischem und/oder abschnittsweise geraden Längsschnitt auf. Der Querschnitt der Ausnehmung 67 nimmt von innen (bezogen auf das ringförmige Bauteil 63) nach außen kontinuierlich zu.

Ein Teil der Drucktaste 60, nämlich sein Bereich nahe dem Rand 62, insbesondere das ringförmige Bauteil 63 der Drucktaste 60, sowie der rohrförmige Bereich 50 am Außenschaft 30 und der Arretierungsschieber 40 sind koaxial angeordnet. Von der Ausnehmung 47, der Öffnung 57, der Ausnehmung 67 und den Gewinden 82, 83 abgesehen sind der Arretierungsschieber 40, der rohrförmige Bereich 50 und die Drucktaste 60 rotationssymmetrisch zur selben Symmetrieachse in der Schnittebene der Figur 3.

Zwischen dem vom Außenschaft 30 abgewandten Ende des rohrförmigen Bereichs 50 und der Innenseite des becherförmigen Bauteils 61 der Drucktaste 60 ist ein Hohlraum vorgesehen, der einerseits eine Bewegung der Drucktaste 60 zu dem Außenschaft 30 hin ermöglicht und andererseits eine Feder 86 zwischen dem becherförmigen Bauteil 61 der Drucktaste 60 und dem rohrförmigen Bereich 50 am Außenschaft 30 enthält. Die Feder 86 schiebt die Drucktaste 60 vom Außenschaft 30 weg. Die Drucktaste 60 kann manuell gegen die elastische Kraft der Feder 86 in eine zweiten Richtung 68 bewegt werden. Die zweite Richtung 68, in der die Drucktaste 60 relativ zum Außenschaft 30 bewegbar ist, ist entgegengesetzt zur ersten Richtung 48, in der der Arretierungsschieber 40 relativ zum Außenschaft 30 bewegbar ist.

Die Arretiereinrichtung 34 weist ferner eine Wippe bzw. einen Hebel 70 zur mechanischen Kopplung der Drucktaste 60 mit dem Arretierungsschieber 40 auf. Der Hebel 70 ist bei dem dargestellten Beispiel ein einfacher Stift mit im Wesentlichen kreiszylindrischer Gestalt. Die Symmetrieachse des Hebels 70 liegt in der Schnittebene der Figur 3. Ein erstes, bei dem dargestellten Beispiel abgerundetes, insbesondere eine halbkugelförmige Gestalt aufweisendes Ende 74 ist in der Ausnehmung 47 im Arretierungsschieber 40 angeordnet. Ein mittlerer Bereich 75 des Hebels 70 ist in der Öffnung 57 im rohrförmigen Bereich 50 angeordnet bzw. greift durch diese Öffnung 57 hindurch. Ein zweites, bei dem dargestellten Beispiel einen rechteckigen Längsschnitt aufweisendes Ende 76 des Hebels 70 ist in der Ausnehmung 67 in der Drucktaste 60 angeordnet.

Die Ausnehmung 47 im Arretierungsschieber 40, die Öffnung 57 im rohrförmigen Bereich 50 am Außenschaft 30 und die Ausnehmung 67 in der Drucktaste 60 sind jeweils so ausgebildet, dass der Hebel 70 bei der in Figur 3 dargestellten Situation bzw. Konfiguration gleichzeitig an zwei einander gegenüberliegenden Punkten in oder nahe der Schnittebene der Figur 3 an der Ausnehmung 47 im Arretierungsschieber 40 anliegt und/oder gleichzeitig an zwei einander gegenüberliegenden Punkten in oder nahe der Schnittebene der Figur 3 am Rand der Öffnung 57 im rohrförmigen Bereich 50 anliegt und/oder gleichzeitig an zwei einander gegenüberliegenden Punkten in oder nahe der Schnittebene der Figur 3 am Rand der Ausnehmung 67 in der Drucktaste 60 anliegt. Auf diese Weise hindert der Hebel 70 die Drucktaste 60 daran, sich vom Außenschaft 30 weiter als in Figur 3 dargestellt zu entfernen. Anders ausgedrückt ist die in Figur 3 dargestellte Position der Drucktaste 60 die am weitesten vom Außenschaft 30 entfernte Position, die von dem Hebel 70 zugelassen wird.

Ausgehend von der in Figur 3 dargestellten Situation bzw. Konfiguration kann die Drucktaste 60 in der zweiten Richtung 68 zum Außenschaft 30 hin bewegt werden. Dabei wird die Ausnehmung 67 in der Drucktaste 60 relativ zur Öffnung 57 im rohrförmigen Bereich 50 verschoben. Dies erzwingt eine Schwenkbewegung des Hebels 70 um eine Schwenkachse 78 orthogonal zur Schnittebene der Figur 3. Die Schwenkachse 78 des Hebels 70 ist nicht durch ein herkömmliches Lager definiert. Der Hebel 70 weist weder eine Welle noch eine Bohrung für eine Welle noch Achszapfen noch eine Schneide noch eine Pfanne noch einen kugelflächenförmigen oder zur Schwenkachse 78 symmetrischen kreiszylindrischen Bereich oder eine andere herkömmliche Einrichtung zur Lagerung auf. Vielmehr ist der mittlere Bereich 75 des Hebels 70 lediglich durch die Öffnung 57 im rohrförmigen Bereich 50 geführt. Dadurch ist die Schwenkachse 78 des Hebels 70 auch nicht so präzise definiert wie bei einem herkömmlichen Lager. Die in Figur 3 dargestellte extreme Konfiguration ist jedoch durch das erwähnte gleichzeitige Anliegen des Hebels an zwei gegenüberliegenden Punkten des Rands der Öffnung 57 und/oder an zwei gegenüberliegenden Punkten an einer der Ausnehmungen 47, 67 eindeutig definiert.

Wenn durch manuellen Druck die Drucktaste 60 ausgehend von der in Figur 3 dargestellten Position zum Außenschaft 30 hin verschoben und der Hebel 70 entsprechend um seine Schwenkachse 78 geschwenkt wird, erzwingt dies eine Bewegung des Arretierungsschiebers 40 in der ersten Richtung 48.

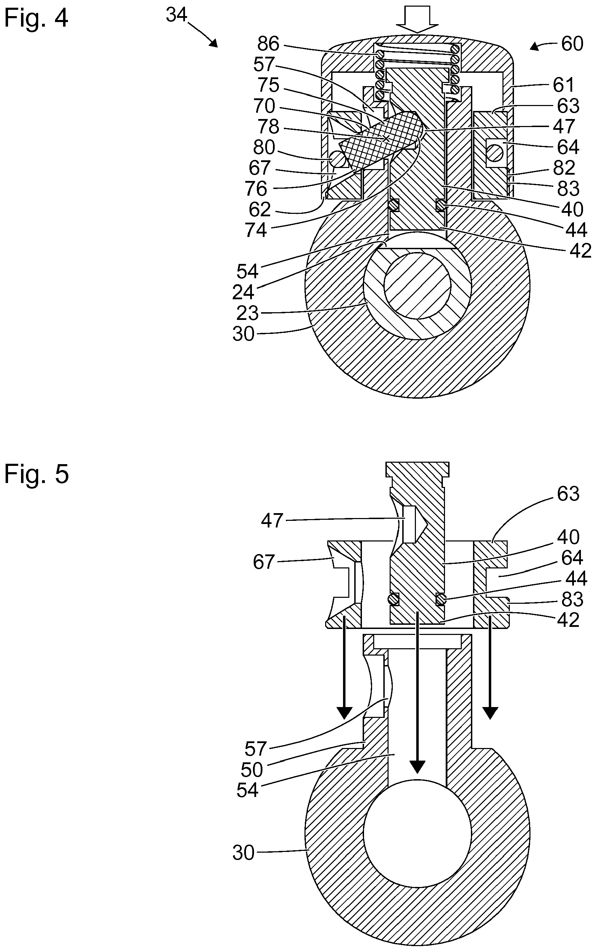

Figur 4 zeigt eine weitere schematische Schnittdarstellung der anhand der Figur 3 dargestellten Arretiereinrichtung 34. Die Art der Darstellung, insbesondere die Schnittebene, entspricht derjenigen der Figur 3.

Figur 4 zeigt eine Situation bzw. Konfiguration, die sich von der anhand der Figur 3 dargestellten dadurch unterscheidet, dass durch manuellen Druck auf die Drucktaste 60 - angedeutet durch einen breiten Pfeil - die Drucktaste 60 zum Außenschaft 30 hin verschoben ist. Insbesondere zeigt Figur 4 eine zu der in Figur 3 gezeigten Position entgegengesetzte extreme Position der Drucktaste 60. Bei der in Figur 4 gezeigten extremen Position der Drucktaste 60 liegt die Drucktaste 60, insbesondere ihr Rand 62 und das ringförmige Bauteil 63, an einer korrespondierenden Fläche am Außenschaft 30 an. Alternativ oder zusätzlich liegt der Hebel gleichzeitig an zwei einander gegenüberliegenden Punkten an der Ausnehmung 47 im Arretierungsschieber 40 und/oder gleichzeitig an zwei einander gegenüberliegenden Punkten am Rand der Öffnung 57 im rohrförmigen Bereich 50 und/oder gleichzeitig an zwei einander gegenüberliegenden Punkten an der Ausnehmung 67 in der Drucktaste 60 an.

Wie bereits erwähnt, erzwingt die Verschiebung der Drucktaste 60 von der in Figur 3 gezeigten Position zu der in Figur 4 gezeigten Position eine Schwenkbewegung des Hebels 70 um seine Schwenkachse 78 orthogonal zu den Zeichenebenen der Figuren 3 und 4. Die Schwenkbewegung des Hebels 70 wiederum erzwingt eine Bewegung des Arretierungsschiebers 40 in der zur Bewegung der Drucktaste 60 entgegengesetzten Richtung, nämlich in der in Figur 3 durch einen Pfeil angedeuteten ersten Richtung 48 relativ zum Außenschaft 30. Der Hebel 70 koppelt somit die Bewegung der Drucktaste 60 mit einer entgegengesetzten Bewegung des Arretierungsschiebers 40. Diese Kopplung ist nicht in jeder Position der Drucktaste 60 so eindeutig wie sie im Falle einer herkömmlichen Lagerung des Hebels 70 wäre. Der Hebel 70 weist zumindest in einem Bereich zwischen den in den Figuren 3 und 4 gezeigten extremen Positionen ein mechanisches Spiel in der Öffnung 57 im rohrförmigen Bereich 50 des Außenschafts 30 auf. Die durch den Hebel 70 vermittelte Kopplung der Bewegung der Drucktaste 60 und des Arretierungsschiebers 40 ist jedoch insbesondere in den in den Figuren 3 und 4 gezeigten extremen Positionen eindeutig. Dazu kann der O-Ring 80 beitragen, der auf das zweite Ende 76 des Hebels 70 einwirkt und dabei das erste Ende 74 des Hebels 70 jederzeit in die Ausnehmung 47 im Arretierungsschieber 40 drückt.

Da der O-Ring 80 in einer Situation bzw. Konfiguration zwischen den in den Figuren 3 und 4 dargestellten extremen Positionen die größte elastische Verformung und damit die größte potenzielle Energie aufweist, kann der O-Ring 80 das taktile Empfinden beim manuellen Druck auf die Drucktaste 60 positiv beeinflussen. Insbesondere können die Feder 86 und der O-Ring 80 zusammen bewirken, dass die zur Bewegung der Drucktaste 60 erforderliche Kraft zu Beginn einer Bewegung von der in Figur 3 gezeigten Position zu der in Figur 4 gezeigten Position zunächst deutlich ansteigt und gegen Ende der Bewegung hin schwächer ansteigt oder sogar wieder leicht abnimmt.

Bei der in Figur 4 gezeigten Situation bzw. Konfiguration ist der Arretierungsschieber 40 vollständig aus der Ausnehmung 24 im Schaft 23 des Endoskops herausgehoben. Deshalb kann in der in Figur 4 gezeigten Situation bzw. Konfiguration der Außenschaft 30 relativ zum Schaft 23 verschoben werden (vgl. Figuren 1 und 2).

Figur 5 zeigt eine schematische Darstellung eines Schnitts durch Teile der anhand der Figuren 3 und 4 dargestellten Arretiereinrichtung. Die Art der Darstellung, insbesondere die Schnittebene, entspricht derjenigen der Figuren 3 und 4.

In Figur 5 ist eine Situation während des Zusammensetzens des medizinischen Instruments oder zumindest der Arretiereinrichtung gezeigt. Insbesondere ist der Arretierungsschieber 40 in einer Position und einer Orientierung relativ zum rohrförmigen Abschnitt 50 am Außenschaft 30 gezeigt, von der aus er unmittelbar durch eine reine geradlinige Translationsbewegung (angedeutet durch den mittleren, längeren Pfeil) in den Hohlraum 54 im rohrförmigen Bereich 50 eingesetzt werden kann.

Das ringförmige Bauteil 63 ist in einer Position und einer Orientierung gezeigt, von der aus es unmittelbar mittels einer geradlinigen Translationsbewegung (in Figur 5 angedeutet durch die beiden äußeren, kürzeren Pfeile) über den rohrförmigen Bereich 50 am Außenschaft 30 gestülpt werden kann. Diese Bewegung entspricht einem Einführen des rohrförmigen Bereichs 50 am Außenschaft 30 in das ringförmige Bauteil 63.

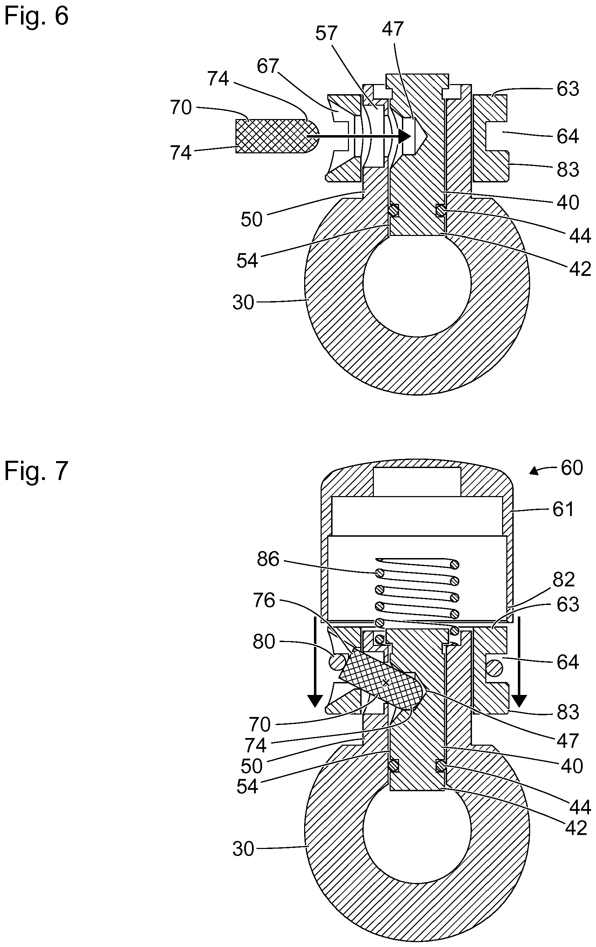

Figur 6 zeigt eine weitere schematische Schnittdarstellung von Teilen der anhand der Figuren 3 und 4 dargestellten Arretiereinrichtung während des Zusammensetzens. Die Art der Darstellung, insbesondere die Schnittebene, entspricht derjenigen der Figuren 3 bis 5.

In Figur 6 ist eine Situation bzw. Konfiguration gezeigt, die der in Figur 5 gezeigten während des Zusammensetzens nachfolgt. Sowohl der Arretierungsschieber 40 als auch das ringförmige Bauteil 63 sind relativ zum rohrförmigen Bereich 50 am Außenschaft 30 so ausgerichtet, dass die Ausnehmung 47 im Arretierungsschieber 40, die Öffnung 57 im rohrförmigen Bereich 50 am Außenschaft 30 und die Ausnehmung 67 im ringförmigen Bauteil 63 fluchten. In dieser ausgerichteten Situation kann der Hebel 70 durch eine geradlinige Translationsbewegung (in Figur 6 angedeutet durch einen Pfeil) eingesetzt werden. Dies wird insbesondere dadurch ermöglicht, dass die Ausnehmung 67 durch eine Durchgangsbohrung im ringförmigen Bauteil 63 gebildet ist, die erst nachfolgend durch das becherförmige Bauteil 61 (vgl. Figuren 3, 4) nach außen hin verschlossen wird, jedoch bei der in Figur 6 dargestellten Situation noch offen ist.

Figur 7 zeigt eine weitere schematische Schnittdarstellung aller Teile der anhand der Figuren 3 und 4 dargestellten Arretiereinrichtung. Die Art der Darstellung, insbesondere die Schnittebene, entspricht derjenigen der Figuren 3 bis 6.

Die in Figur 7 gezeigte Situation bzw. Konfiguration folgt beim Zusammensetzen nach der in Figur 6 gezeigten. Der O-Ring 80 ist in die außen umlaufende Nut 64 am ringförmigen Bauteil 63 eingelegt, liegt am zweiten Ende 76 des Hebels 70 an und drückt das erste Ende 74 des Hebels 70 in die Ausnehmung 47 im Arretierungsschieber 40. Die als Schraubenfeder ausgebildete Feder 86 ist auf dem rohrförmigen Bereich 50 am Außenschaft 30 aufgesetzt.

Das becherförmige Bauteil 61 ist in Figur 7 in einer Position und Orientierung dargestellt, von der aus es durch eine geradlinige Translationsbewegung (in Figur 7 angedeutet durch zwei Pfeile) über das ringförmige Bauteil 63 gestülpt werden kann. Zum Ende dieser Translationsbewegung hin muss diese mit einer Rotation des Becherbauteils 61 um seine Symmetrieachse einhergehen, um das Innengewinde 82 am becherförmigen Bauteil 61 mit dem Außengewinde 83 am ringförmigen Bauteil 63 zu verbinden. Alternativ oder zusätzlich zur Verschraubung können das becherförmige Bauteil 61 und das ringförmige Bauteil 63 durch Schweißen, Löten oder Kleben oder auf andere form-, stoff- oder kraftschlüssige Weise verbunden werden.

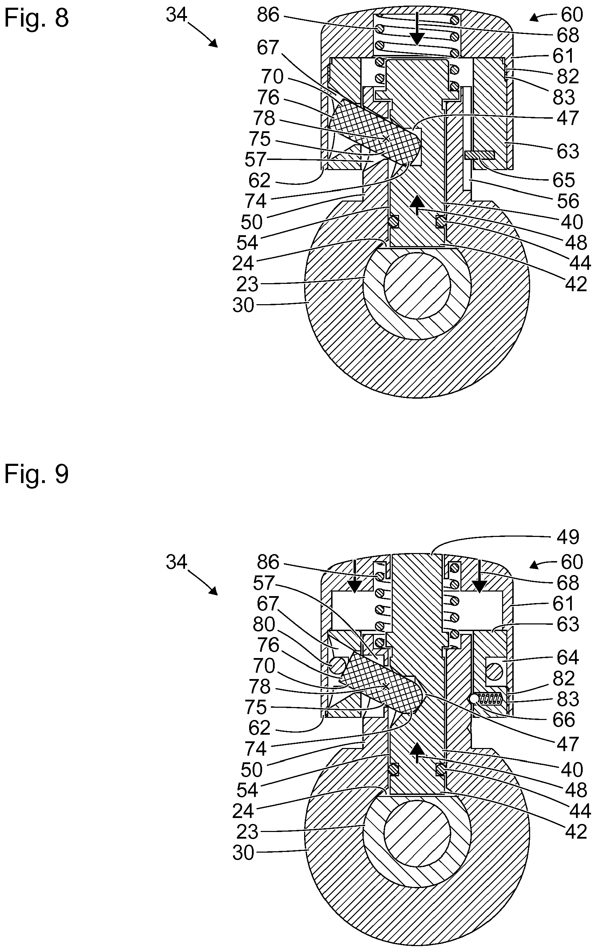

Figur 8 zeigt eine schematische Schnittdarstellung einer alternativen Ausgestaltung einer Arretiereinrichtung 34, die beispielsweise für das anhand der Figuren 1 und 2 dargestellte medizinische Instrument 10 verwendbar ist. Die Art der Darstellung, insbesondere die Schnittebene, entspricht derjenigen der Figuren 3 bis 7.

Die in Figur 8 gezeigte Arretiereinrichtung 34 ähnelt in einigen Merkmalen, Eigenschaften und Funktionen der anhand der Figuren 3 und 4 dargestellten Arretiereinrichtung und ist mittels Verfahrensschritten, die den anhand der Figuren 5 bis 7 dargestellten, ähneln, zusammensetzbar. Nachfolgend sind Merkmale, Eigenschaften und Funktionen beschrieben, durch die die in Figur 8 gezeigte Arretiereinrichtung 34 sich von der anhand der Figuren 3 und 4 dargestellten unterscheidet. Die Merkmale und Eigenschaften, in denen die in Figur 8 gezeigte Arretiereinrichtung 34 sich von der anhand der Figuren 3 und 4 dargestellten Arretiereinrichtung unterscheidet, können einzeln mit den Merkmalen und Eigenschaften der anhand der Figuren 3 und 4 dargestellten Arretiereinrichtung kombiniert werden.

Ein Unterschied zwischen der in Figur 8 gezeigten Arretiereinrichtung 34 und der anhand der Figuren 3 und 4 dargestellten Arretiereinrichtung besteht darin, dass kein O-Ring vorgesehen ist, um den Hebel 70 jederzeit ganz in die Ausnehmung 47 im Arretierungsschieber 40 zu schieben. Stattdessen weist der Hebel 70 eine größere Länge und gekrümmte Oberflächen an den Enden 74, 76 auf. Die Oberflächen an den Enden 74, 76 des Hebels 70 sind insbesondere Ausschnitte von Kugelflächen, deren Mittelpunkte zusammenfallen. Beispielsweise sind die Oberflächen an den Enden 74, 76 des Hebels 70 Ausschnitte einer Kugelfläche oder Ausschnitte zweier Kugelflächen unterschiedlicher Radien, deren Mittelpunkte zusammenfallen und beispielsweise auf der Schwenkachse 78 des Hebels 70 liegen. Alternativ können die Oberflächen an den Enden 74, 76 des Hebels 70 Ausschnitte von Oberflächen von Kreiszylindern, deren Zylinderachsen zusammenfallen, sein. Insbesondere fallen die Zylinderachsen mit der Schwenkachse 78 des Hebels 70 zusammen.

Durch diese Ausgestaltung der Oberflächen an den Enden 74, 76 des Hebels 70 kann der Hebel 70 innerhalb eines Winkelbereichs, der vom Durchmesser des Hebels 70 abhängt, spiel- und reibungsarm zwischen der in diesem Fall ebenen Stirnwand der Ausnehmung 47 im Arretierungsschieber 40 und der inneren Oberfläche des becherförmigen Bauteils 61 geführt sein.

Ein weiterer Unterschied besteht darin, dass bei der in Figur 8 gezeigten Arretiereinrichtung 34 das Innengewinde 82 am becherförmigen Bauteil 61 und das korrespondierende Außengewinde 83 am ringförmigen Bauteil 63 anders angeordnet sind, nämlich nahe dem vom Rand 62 des becherförmigen Bauteils 61 abgewandten Rand des ringförmigen Bauteils 63. Der O-Ring 80 (im Falle einer Ausgestaltung des Hebels 70 entsprechend der Darstellung anhand der Figuren 3 und 4) bzw. der Hebel 70 selbst (im Falle seiner Ausgestaltung entsprechend der Darstellung anhand der Figur 8) kommt deshalb auch während des Zusammensetzens nicht mit dem Innengewinde 82 am becherförmigen Bauteil 61 in Berührung.

Ein weiterer Unterschied besteht darin, dass bei der in Figur 8 gezeigten Arretiereinrichtung 34 die Öffnung 57 im rohrförmigen Bereich 50 am Außenschaft 30 überwiegend konisch ausgebildet ist. Sowohl die Ausnehmung 47 im Arretierungsschieber 40 als auch die Öffnung 57 im rohrförmigen Bereich 50 am Außenschaft 30 als auch die Ausnehmung 67 in der Drucktaste 60 können jeweils abschnittsweise, weitgehend oder vollständig konisch ausgebildet sein oder im Längsschnitt abschnittsweise rechteckig ausgebildet sein bzw. stufenförmig variierende Querschnitte aufweisen.

Eine Ausgestaltung derart, dass der ringförmige Bereich minimalen Querschnitts möglichst schmal ausgebildet ist (wie dies auch bei allen hier beschriebenen Ausführungsbeispielen für die Öffnung 57 im rohrförmigen Bereich 50 am Außenschaft 30 und für die Ausnehmung 67 in der Drucktaste 60 gilt) ist vorteilhaft, weil sie bei gegebenem maximalen Schwenkwinkel des Hebels 70 das Spiel des Hebels bei Positionen zwischen den in den Figuren 3, 4 und 8 gezeigten extremen Positionen minimiert. Zur Förderung des gleichen Ziels taucht der die Gestalt des Hebels 70 zwischen seinen Enden 74, 76 dominierende zylindrische Teil des Hebels möglichst wenig in den zylindrischen Abschnitt der Ausnehmung 47 im Arretierungsschieber 40 ein.

Ein weiterer Unterschied besteht darin, dass die in Figur 8 gezeigte Arretiereinrichtung einen Stift 65 am ringförmigen Bauteil 63 aufweist, der nach innen bzw. in den vom ringförmigen Bauteil 63 umschlossenen Raumbereich hin ragt. Der Stift 65 am ringförmigen Bauteil 63 greift in eine korrespondierende Nut 56 im rohrförmigen Bereich 50 am Außenschaft 30 ein. Die Nut 56 ist gerade und parallel zur zweiten Richtung 68, in der die Drucktaste 60 ausgehend von ihrer in Figur 8 gezeigten Position bewegbar ist. Der Stift 65 am ringförmigen Bauteil 63 und die Nut 56 im rohrförmigen Bereich 50 am Außenschaft 30 werden jeweils von der Schnittebene der Figur 8 geschnitten, können jedoch alternativ an anderen Positionen vorgesehen sein. Die Nut 56 ist so lang, dass das ringförmige Bauteil 63, wie anhand der Figur 5 dargestellt, über den rohrförmigen Bereich 50 gestülpt werden kann.

Der Eingriff des Stifts 65 am ringförmigen Bauteil 63 in die Nut 56 im rohrförmigen Bereich 50 am Außenschaft 30 unterbindet eine Rotation des ringförmigen Bauteils 63 und damit auch der gesamten Drucktaste 60 relativ zum rohrförmigen Bereich 50 am Außenschaft 30. Eine solche Rotation kann zwar auch durch den Hebel 70 unterbunden werden. Der Eingriff des Stifts 65 am ringförmigen Bauteil 63 in die Nut 56 am rohrförmigen Bereich 50 ermöglicht jedoch eine präzisere Führung und entlastet den Hebel 70 von dieser sekundären Aufgabe.

Ein weiterer Unterschied besteht darin, dass bei der in Figur 8 gezeigten Arretiereinrichtung 34 die Feder 86 nicht zwischen dem rohrförmigen Bereich 50 am Außenschaft 30 und der Drucktaste 60, sondern zwischen dem Arretierungsschieber 40 und der Drucktaste 60 angeordnet ist. Ferner sind das der Feder 86 zugewandte Ende des Arretierungsschiebers 40 und das becherförmige Bauteil 61 so ausgebildet, dass die Feder 86 nahezu über ihre gesamte Länge jederzeit zumindest entweder vom Arretierungsschieber 40 oder vom becherförmigen Bauteil 61 geführt ist.

Figur 9 zeigt eine schematische Schnittdarstellung einer alternativen Ausgestaltung einer Arretiereinrichtung 34, die beispielsweise für das anhand der Figuren 1 und 2 dargestellte medizinische Instrument 10 verwendbar ist. Die Art der Darstellung, insbesondere die Schnittebene, entspricht derjenigen der Figuren 3 bis 8.

Die in Figur 9 gezeigte Arretiereinrichtung 34 ähnelt in einigen Merkmalen, Eigenschaften und Funktionen den anhand der Figuren 3 bis 8 dargestellten Arretiereinrichtungen und ist mittels Verfahrensschritten, die den anhand der Figuren 5 bis 7 dargestellten, ähneln, zusammensetzbar. Nachfolgend sind Merkmale, Eigenschaften und Funktionen beschrieben, durch die die in Figur 9 gezeigte Arretiereinrichtung 34 sich von den anhand der Figuren 3 bis 8 dargestellten unterscheidet. Die Merkmale und Eigenschaften, in denen die in Figur 9 gezeigte Arretiereinrichtung 34 sich von den anhand der Figuren 3 bis 8 dargestellten Arretiereinrichtungen unterscheidet, können einzeln mit den Merkmalen und Eigenschaften der anhand der Figuren 3 bis 8 dargestellten Arretiereinrichtungen kombiniert werden.

Ein Unterschied zwischen der in Figur 9 gezeigten Arretiereinrichtung 34 und den anhand der Figuren 3 bis 8 dargestellten Arretiereinrichtung besteht darin, dass das becherförmige Bauteil 61 der Drucktaste 60 eine Öffnung aufweist, in der ein Ende 49 des Arretierungsschiebers 40 angeordnet und von außen sichtbar ist. Das in der Öffnung in dem becherförmigen Bauteil 61 sichtbare Ende 49 des Arretierungsschiebers 40 ist das zu dem Wirkende 42 des Arretierungsschiebers 40 entgegengesetzte, d. h. von diesem abgewandte Ende.

Bei der in Figur 9 gezeigten Situation bzw. Konfiguration befindet der Arretierungsschieber 40 sich in seiner vorgesehenen Wirkposition, die formschlüssig definiert ist. Das Wirkende 42 des Arretierungsschiebers 40 greift in die Ausnehmung 24 in dem Endoskop ein. Bei dieser Wirkposition des Arretierungsschiebers ist das sichtbare Ende 49 des Arretierungsschiebers bündig mit dem umgebenden Bereich des becherförmigen Bauteils 61. Daran ist die Wirkposition des Arretierungsschiebers sowohl visuell als auch taktil schnell und klar erkennbar.

Die in Figur 9 gezeigte Arretiereinrichtung 34 unterscheidet sich von den anhand der Figuren 3 bis 8 dargestellten Arretiereinrichtungen ferner dadurch, dass eine Rasteinrichtung 66 zwischen der Drucktaste 60 und dem rohrförmigen Bereich 50 an dem Außenschaft 30 vorgesehen ist. Diese Rasteinrichtung 66 ist beispielhaft durch eine Kugel, die durch eine Feder in eine von zwei Ausnehmungen an dem rohrförmigen Bereich 50 an dem Außenschaft 30 gedrückt wird, angedeutet.

Die Rasteinrichtung 66 bewirkt ein Verharren des Arretierungsschiebers 40 und der Drucktaste 60 in ihren in Figur 9 gezeigten Positionen auch dann, wenn kleine Kräfte auf sie wirken. Um die Drucktaste 60 und damit auch den Arretierungsschieber 40 aus ihren in Figur 9 gezeigten Positionen auszulenken, müssen die Kraft der Feder 86 und die Rastkraft der Rasteinrichtung 66 überwunden werden.

Ausgehend von der in Figur 9 dargestellten Situation bzw. Konfiguration kann die Drucktaste 60 in der zweiten Richtung 68 zum Außenschaft 30 hin bewegt werden. Wie bereits anhand der Figuren 3 und 4 dargestellt, geht eine Bewegung der Drucktaste 60 in der zweiten Richtung 68 mit einer Schwenkbewegung des Hebels 70 und einer Bewegung des Arretierungsschiebers 40 in der ersten Richtung 48 einher.

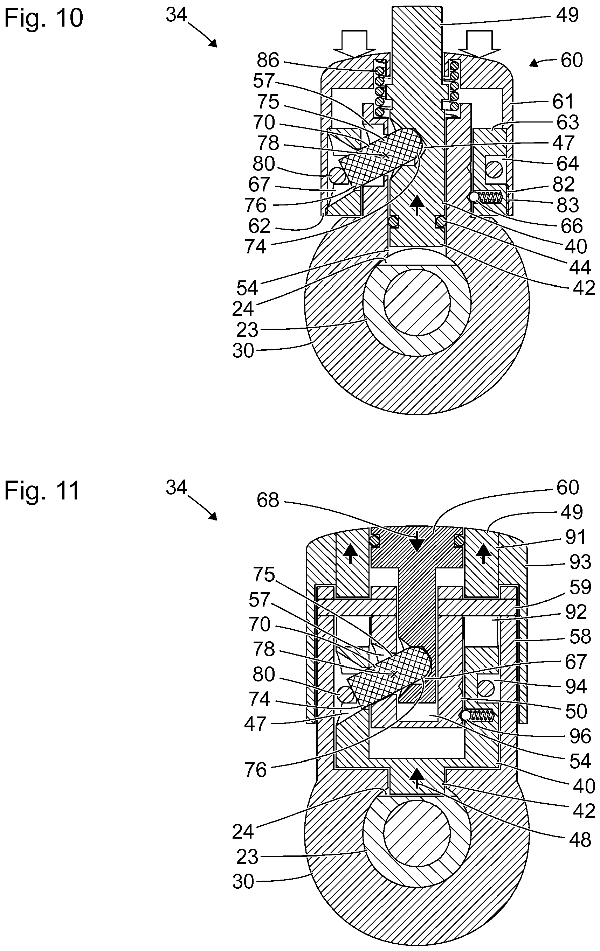

Figur 10 zeigt eine weitere schematische Schnittdarstellung der anhand der Figur 9 dargestellten Arretiereinrichtung 34. Die Art der Darstellung, insbesondere die Schnittebene, entspricht derjenigen der Figur 9.

Figur 10 zeigt eine Situation bzw. Konfiguration, die sich von der anhand der Figur 9 dargestellten dadurch unterscheidet, dass durch manuellen Druck auf die Drucktaste 60 - angedeutet durch breite Pfeile - die Drucktaste 60 zum Außenschaft 30 hin verschoben ist. Insbesondere zeigt Figur 10 eine zu der in Figur 9 gezeigten Position entgegengesetzte extreme Position der Drucktaste 60. Bei der in Figur 10 gezeigten extremen Position der Drucktaste 60 liegt die Drucktaste 60, insbesondere ihr Rand 62 und das ringförmige Bauteil 63, an einer korrespondierenden Fläche am Außenschaft 30 an. Alternativ oder zusätzlich liegt der Hebel gleichzeitig an zwei einander gegenüberliegenden Punkten an der Ausnehmung 47 im Arretierungsschieber 40 und/oder gleichzeitig an zwei einander gegenüberliegenden Punkten am Rand der Öffnung 57 im rohrförmigen Bereich 50 und/oder gleichzeitig an zwei einander gegenüberliegenden Punkten an der Ausnehmung 67 in der Drucktaste 60 an.

Wie bereits anhand der Figuren 3 und 4 dargestellt erzwingt die Verschiebung der Drucktaste 60 von der in Figur 9 gezeigten Position zu der in Figur 10 gezeigten Position eine Schwenkbewegung des Hebels 70 um seine Schwenkachse 78 orthogonal zu den Zeichenebenen der Figuren 9 und 10 und eine Bewegung des Arretierungsschiebers 40 in der zu der Bewegung der Drucktaste 60 entgegengesetzten ersten Richtung 48.

Alternativ und abweichend von der Darstellung anhand der Figuren 9 und 10 kann die Feder 86 entfallen. In diesem Fall kann der Arretierungsschieber 40 durch manuellen Druck auf das sichtbare Ende 49 in seine in Figur 9 gezeigte Wirkposition bewegt werden, und durch manuellen Druck auf die Drucktaste 60 kann die Arretiereinrichtung 40 aus ihrer Wirkposition heraus in die in Figur 10 gezeigte Position bewegt werden.

Ferner kann die Rasteinrichtung 66 abweichend von der Darstellung in den Figuren 9 und 10 nur eine Vertiefung an dem rohrförmigem Bereich 50 an dem Außenschaft 30 aufweisen. Wenn die Arretiereinrichtung 34 die Feder 86 aufweist, weist die Rasteinrichtung 66 insbesondere nur diejenige Vertiefung auf, die die Drucktaste 60 in der in Figur 10 gezeigten Position fixiert.

Alternativ und abweichend von der Darstellung anhand der Figuren 9 und 10 kann die Rasteinrichtung 66 ganz entfallen.

Figur 11 zeigt eine schematische Schnittdarstellung einer alternativen Ausgestaltung einer Arretiereinrichtung 34, die beispielsweise für das anhand der Figuren 1 und 2 dargestellte medizinische Instrument 10 verwendbar ist. Die Art der Darstellung, insbesondere die Schnittebene, entspricht derjenigen der Figuren 3 bis 10.

Die in Figur 11 gezeigte Arretiereinrichtung 34 ähnelt in einigen Merkmalen, Eigenschaften und Funktionen den anhand der Figuren 3 bis 10 dargestellten Arretiereinrichtungen und ist mittels Verfahrensschritten, die den anhand der Figuren 5 bis 7 dargestellten, ähneln, zusammensetzbar. Nachfolgend sind Merkmale, Eigenschaften und Funktionen beschrieben, durch die die in Figur 11 gezeigte Arretiereinrichtung 34 sich von den anhand der Figuren 3 bis 10 dargestellten unterscheidet. Die Merkmale und Eigenschaften, in denen die in Figur 11 gezeigte Arretiereinrichtung 34 sich von den anhand der Figuren 3 bis 10 dargestellten Arretiereinrichtungen unterscheidet, können teilweise einzeln mit den Merkmalen und Eigenschaften der anhand der Figuren 3 bis 10 dargestellten Arretiereinrichtungen kombiniert werden.

Ähnlich wie bei der anhand der Figuren 9 und 10 dargestellten Arretiereinrichtung weist auch bei der in Figur 11 gezeigten Arretiereinrichtung 34 der Arretierungsschieber 40 ein sichtbares Ende 49 auf. Bei beiden Beispielen sind das sichtbare Ende 49 des Arretierungsschiebers 40 und die Drucktaste 60 konzentrisch angeordnet. Im Unterschied zu der anhand der Figuren 9 und 10 dargestellten Arretiereinrichtung ist jedoch bei der in Figur 11 gezeigten Arretiereinrichtung 34 nicht das sichtbare Ende 49 des Arretierungsschiebers 40 von der Drucktaste 60, sondern die Drucktaste 60 von dem sichtbare Ende des Arretierungsschiebers 40 umgeben.

Die Drucktaste 60 weist bei der in Figur 11 gezeigten Arretiereinrichtung 34 eine im Wesentlichen stiftförmige Gestalt auf. Bei dem dargestellten Beispiel weist das sichtbare Ende der Drucktaste 60 einen vergrößerten Durchmesser auf.

Die Drucktaste 60 ist teilweise in einem rohrförmigen Bereich 50 an dem Außenschaft 30 des medizinischen Instruments angeordnet. Bei dem in Figur 11 gezeigten Beispiel ist das rohrförmige Bereich 50 an einem Ende, nämlich an dem von dem sichtbaren Ende der Drucktaste 60 abgewandten und dem Außenschaft 34 zugewandten Ende, geschlossen, weist also eine insgesamt becherförmige Gestalt auf.

Der rohrförmige Bereich 50 ist durch ein Bauteil gebildet, das über mehrere Stifte 59 mit einem weiteren rohrförmigen Bereich 58 mechanisch starr verbunden ist. Der weitere rohrförmige Bereich 58 ist unmittelbar mit dem Außenschaft 34 verbunden und kann mit diesem einstückig oder monolithisch ausgebildet sein.

Der Arretierungsschieber 40 ist weitgehend durch ein im Wesentlichen becherförmiges Bauteil 91 gebildet. Das Wirkende 42 ist am Boden des becherförmigen Bauteils 91 vorgesehen. Der Rand des becherförmigen Bauteils 91 bildet einen Teil des sichtbaren Endes 49 des Arretierungsschiebers 40 und umschließt die Drucktaste 60 ringförmig. Ein Bereich des becherförmigen Bauteils 91 zwischen Boden und Rand, der im Wesentlichen die Gestalt einer Mantelfläche eines Kreiszylinders aufweist, ist teilweise in einem ringförmigen Spalt zwischen dem rohrförmigen Bereich 50 und dem weiteren rohrförmigen Bereich 58 an dem Außenschaft 34 angeordnet und weist mehrere Schlitze 92 auf. Jeder Schlitz 92 erstreckt sich in einer Richtung parallel zu der Richtung 48, in der der Arretierungsschieber 40 bewegbar ist. Jeder Schlitz 92 wird von einem Stift 59 durchdrungen, der den rohrförmigen Bereich 50 innerhalb des becherförmigen Bauteils 91 mit dem weiteren rohrförmigen Bereich 58 außerhalb des becherförmigen Bauteils 91 mechanisch starr verbindet.

Der Arretierungsschieber 40 umfasst ferner ein rohrförmiges Bauteil 93, das mit dem Rand des becherförmigen Bauteils 91 mechanisch starr verbunden, insbesondere durch Laserschweißen gefügt ist. Das rohrförmige Bauteils 93 umschließt den weiteren rohrförmigen Bereich 58 an dem Außenschaft 34 und bildet zusammen mit dem Rand des becherförmigen Bauteils 91 das sichtbare Ende 49 des Arretierungsschiebers 40.

Ähnlich wie bei den anhand der Figuren 3 bis 10 dargestellten Arretierungseinrichtungen durchdringt ein Hebel 70 eine Öffnung 57 in dem rohrförmigen Bereich 50 an dem Außenschaft 30. Die Öffnung 57 definiert formschlüssig eine Schwenkachse 78 in einem mittleren Bereich 75 des Hebels 70, um die der Hebel 70 schwenkbar ist. Ein erstes Ende 74 des Hebels 70 greift in eine Öffnung 47 in dem becherförmigen Bauteil 91 des Arretierungsschiebers 40 ein. Ein zweites Ende 76 des Hebels 70 greift in eine Ausnehmung 67 in der Drucktaste 60 ein.

Ein O-Ring 80 aus einem elastischen Material ist in einer Nut 94 in dem becherförmigen Bauteil 91 des Arretierungsschiebers 40 angeordnet. Die Nut 94 schneidet die Öffnung 47 in dem becherförmigen Bauteil 91. Im Bereich der Öffnung 47 liegt der O-Ring 80 an dem ersten Ende 74 des Hebels 70 an. Der O-Ring 80 übt eine Kraft auf den Hebel 70 aus, die dessen zweites Ende 76 in die Ausnehmung 67 in der Drucktaste 60 drückt.

Zwischen dem becherförmigen Bauteil 91 des Arretierungsschiebers 40 und dem rohrförmigen Bereich 50 an dem Außenschaft 34 ist eine Rasteinrichtung 96 vorgesehen. Die Rasteinrichtung definiert zwei Positionen des Arretierungsschiebers 40 relativ zu dem rohrförmigen Bereich 50, in denen die der Arretierungsschieber 40 einrastet. Die Rasteinrichtung 96 ist beispielhaft als federbelastete Kugel, die alternativ in eine von zwei kleinen Ausnehmungen an dem rohrförmigen Bereich 50 an dem Außenschaft 34 eingreift, dargestellt.

Alternativ oder zusätzlich zu der Rasteinrichtung 96 kann - ähnlich wie bei den anhand der Figuren 3 bis 10 dargestellten Arretiereinrichtungen - eine Feder vorgesehen sein, die den Arretierungsschieber 40 in die in Figur 11 gezeigte Wirkposition schiebt.

Bei der in Figur 11 gezeigten Arretiereinrichtung 34 sind die sichtbaren Enden des Arretierungsschiebers 40 und der Drucktaste 60 bündig bzw. fluchtend angeordnet, wenn der Arretierungsschieber 40 seine in Figur 11 gezeigte Wirkposition einnimmt. Deshalb ist sowohl visuell als auch taktil schnell und klar erkennbar, wenn der Arretierungsschieber 40 seine Wirkposition einnimmt.

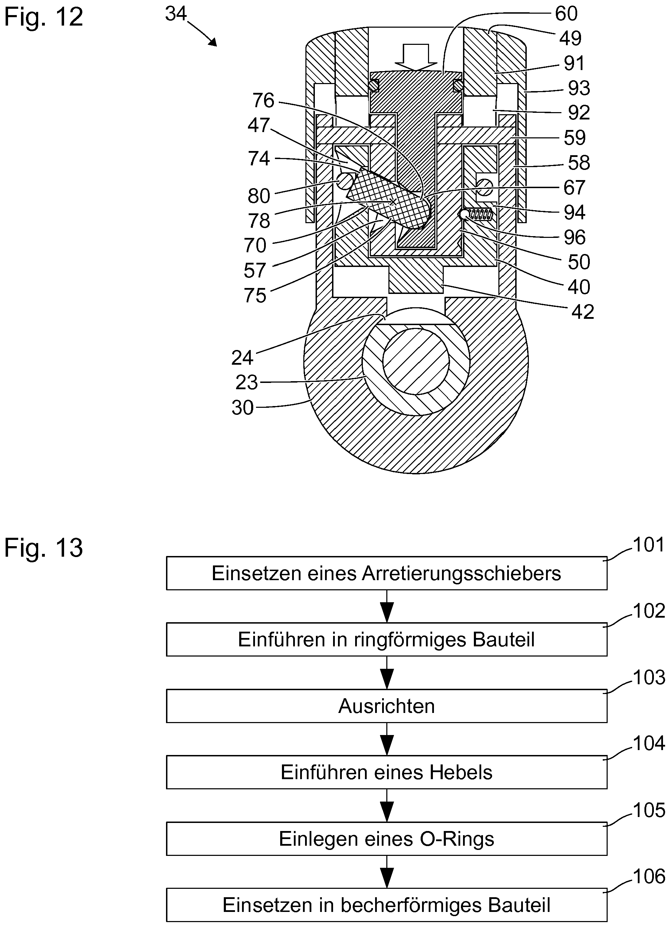

Figur 12 zeigt eine weitere schematische Schnittdarstellung der anhand der Figur 11 dargestellten Arretiereinrichtung 34. Die Art der Darstellung, insbesondere die Schnittebene, entspricht derjenigen der Figur 11.

Figur 12 zeigt eine Situation bzw. Konfiguration, die sich von der anhand der Figur 11 dargestellten dadurch unterscheidet, dass durch manuellen Druck auf die Drucktaste 60 - angedeutet durch einen breiten Pfeil - die Drucktaste 60 zum Außenschaft 30 hin verschoben ist. Insbesondere zeigt Figur 12 eine formschlüssig definierte und zu der in Figur 11 gezeigten Position entgegengesetzte extreme Position der Drucktaste 60.

Wie bereits anhand der Figuren 3, 4, 9 und 10 dargestellt erzwingt die Verschiebung der Drucktaste 60 von der in Figur 11 gezeigten Position zu der in Figur 12 gezeigten Position eine Schwenkbewegung des Hebels 70 um seine Schwenkachse 78 orthogonal zu den Zeichenebenen der Figuren 11 und 12 und eine Bewegung des Arretierungsschiebers 40 in der zu der Bewegung der Drucktaste 60 entgegengesetzten ersten Richtung 48.

Zum Bewegen des Arretierungsschiebers 40 aus der in Figur 11 gezeigten Wirkposition heraus in die in Figur 12 gezeigte Position muss die Drucktaste 60 in die in Figur 12 gezeigte, gegenüber dem sichtbaren Ende 49 des Arretierungsschiebers 40 versenkte Position bewegt werden. Dies schließt eine unbeabsichtigte Betätigung der Drucktaste 60 weitgehend aus.

Figur 13 zeigt ein schematisches Flussdiagramm eines Verfahrens zum Zusammensetzen eines medizinischen Instruments, insbesondere einer Arretiereinrichtung an einem medizinischen Instrument.

Bei einem ersten Schritt 101 wird ein Arretierungsschieber 40 oder eine andere Wirkeinrichtung in einen rohrförmigen Bereich 50, insbesondere in einen Hohlraum 54 in einem rohrförmigen Bereich 50 des medizinischen Instruments 10 eingesetzt. Bei einem zweiten Schritt 102 wird der rohrförmige Bereich 50 in ein ringförmiges Bauteil 63 eingeführt bzw. das ringförmige Bauteil 63 über den rohrförmigen Bereich 50 gestülpt. Der erste Schritt 101 und der zweite Schritt 102 können in der dargestellten Reihenfolge oder gleichzeitig oder in der umgekehrten Reihenfolge ausgeführt werden.

Bei einem dritten Schritt 103 werden die Wirkeinrichtung 40 und das ringförmige Bauteil 63 relativ zu dem rohrförmigen Bereich 50 so ausgerichtet, dass eine Ausnehmung 67 in dem ringförmigen Bauteil 63, eine Öffnung 57 im rohrförmigen Bereich 50 und eine Ausnehmung 47 im Arretierungsschieber 40 fluchten. Der dritte Schritt 103 kann in zwei Teilschritten bereits gleichzeitig mit dem ersten Schritt 101 und/oder mit dem zweiten Schritt 102 ausgeführt werden.

Bei einem vierten Schritt 104 wird ein Hebel 70 in die Ausnehmung 67 in dem ringförmigen Bauteil 63, in die Öffnung 57 im rohrförmigen Bereich 50 und in die Ausnehmung 47 in der Wirkeinrichtung 40 eingeführt.

Bei einem optionalen fünften Schritt 105 wird ein O-Ring 80 in eine ringförmig am äußeren Umfang des ringförmigen Bauteils 63 umlaufende Nut 64 eingesetzt.

Bei einem sechsten Schritt 106 wird das ringförmige Bauteil 63 in ein becherförmiges Bauteil 61 eingesetzt bzw. das becherförmige Bauteil 61 über das ringförmige Bauteil 63 gestülpt. Der sechste Schritt 106 umfasst insbesondere eine dauerhafte mechanische Verbindung des ringförmigen Bauteils 63 mit dem becherförmigen Bauteil 61 durch Schrauben und/oder auf andere form-, stoff- und/oder kraftschlüssige Weise. Der sechste Schritt 106 geht insbesondere einher mit einem Verschließen einer die Ausnehmung 67 in dem ringförmigen Bauteil 63 bildenden Durchgangsbohrung an ihrem radial äußeren Ende durch das becherförmige Bauteil 63. Damit ist der Hebel 70 in der Ausnehmung 47 in der Wirkeinrichtung 40, in der Öffnung 57 im rohrförmigen Bereich 50 und in der Ausnehmung 67 im ringförmigen Bauteil 63 eingeschlossen.

### Bezugszeichen

- 10: medizinisches Instrument, insbesondere Hysteroskop
- 20: Endoskop oder anderes medizinisches Instrument
- 21: proximales Ende des Endoskops 20
- 22: distales Ende des Endoskops 20
- 23: Schaft des Endoskops 20
- 24: Ausnehmung im Endoskop 20
- 30: Außenschaft des medizinischen Instruments 10
- 32: zylindrischer Hohlraum im Außenschaft zur Aufnahme des Endoskops 20
- 34: Arretiereinrichtung für den Außenschaft 30
- 40: Arretierungsschieber als Wirkeinrichtung
- 42: Wirkende des Arretierungsschiebers 40 zum Eingriff in eine Ausnehmung 24 im Endoskop 20
- 44: O-Ring am Arretierungsschiebers 40
- 47: Ausnehmung im Arretierungsschieber 40, insbesondere Sackloch
- 48: erste Richtung, in der der Arretierungsschieber 40 relativ zu dem rohrförmigen Bereich 50 verschiebbar ist
- 49: sichtbares, zum Wirkende 42 entgegengesetztes Ende des Arretierungsschiebers 40
- 50: rohrförmiger Bereich am Außenschaft 30 des medizinischen Instruments 10
- 54: Bohrung bzw. von dem rohrförmigen Bereich 50 umgebener Hohlraum
- 56: Nut im rohrförmigen Bereich 50 parallel zur zweiten Bewegungsrichtung 68
- 57: Öffnung im rohrförmigen Bereich 50 am Außenschaft 30
- 58: weiterer rohrförmiger Bereich an dem Außenschaft 30 des medizinischen Instruments 30
- 59: Stift zwischen dem rohrförmigen Bereich 50 und dem weiteren rohrförmigen Bereich 58
- 60: Drucktaste
- 61: becherförmiges Bauteil der Drucktaste 60
- 62: Rand des becherförmigen Bauteils 61 der Drucktaste 60, eine Öffnung des becherförmigen Bauteils 61 umgebend
- 63: ringförmiges Bauteil der Drucktaste 60
- 64: ringförmig außen umlaufende Nut am ringförmigen Bauteil 63
- 65: Stift im ringförmigen Bauteil 63 der Drucktaste 60, in die Nut 56 im rohrförmigen Bereich 50 eingreifend
- 66: Rasteinrichtung an der Drucktaste 60
- 67: Ausnehmung in der Drucktaste 60
- 68: zweite Richtung, in der die Drucktaste 60 relativ zu dem rohrförmigen Bereich 50 verschiebbar ist
- 70: Hebel zur mechanischen Kopplung der Drucktaste 60 mit dem Arretierungsschieber 40
- 74: erstes Ende des Hebels 70, in die Ausnehmung 47 im Arretierungsschieber 40 eingreifend
- 75: mittlerer Bereich des Hebels 70, durch die Öffnung 57 im rohrförmigen Bereich 50 hindurch greifend
- 76: zweites Ende des Hebels 70, in die Ausnehmung 67 in der Drucktaste 60 eingreifend
- 78: Schwenkachse des Hebels 70, definiert durch die Anordnung des mittleren Bereichs 75 des Hebels 70 in der Öffnung 57 im rohrförmigen Bereich 50
- 80: O-Ring aus einem elastischen Material
- 82: Innengewinde am Rand 62 des becherförmigen Bauteils 61 der Drucktaste 60
- 83: Außengewinde am ringförmigen Bauteil 63 der Drucktaste 60
- 86: Feder zwischen dem rohrförmigen Bereich 50 am Außenschaft 30 oder am Arretierungsschieber 40 einerseits und der Drucktaste 60 andererseits
- 91: becherförmiges Bauteil des Arretierungsschiebers 40, unter anderem das Wirkende 42 des Arretierungsschiebers 40 bildend
- 92: Schlitz in dem becherförmigen Bauteil 91 des Arretierungsschiebers 40
- 93: rohrförmiges Bauteil des Arretierungsschiebers 40, den zweiten rohrförmigen Bereich 58 am Außenschaft teilweise umschließend
- 94: Nut in dem becherförmigen Bauteil 91, den O-Ring 80 aufnehmend
- 96: Rasteinrichtung an dem becherförmigen Bauteil 91 der Drucktaste 60
- 101: Einsetzen eines Arretierungsschiebers 40 in einen rohrförmigen Bereich 50
- 102: Einführen des rohrförmigen Bereichs 50 in ein ringförmiges Bauteil 63
- 103: Ausrichten des Arretierungsschiebers 40 und des ringförmigen Bauteils 63 relativ zu dem rohrförmigen Bereich 50
- 104: Einführen eines Hebels 70 in die Ausnehmung 67 in dem ringförmigen Bauteil 63, in eine Öffnung 57 im rohrförmigen Bereich 50 und in eine Ausnehmung 47 in Arretierungsschieber 40
- 105: Einlegen eines O-Rings 80 in eine ringförmig am äußeren Umfang des ringförmigen Bauteils 63 umlaufende Nut 64
- 106: Einsetzen des ringförmigen Bauteils 63 in ein becherförmiges Bauteil 61

## Patentansprüche

1. Medizinisches Instrument (10), mit:
einer Wirkeinrichtung (40), die in einer ersten Richtung (48) aus einer ersten Wirkposition in eine zweite Wirkposition bewegbar ist;
einer Drucktaste (60), die manuell in einer zweiten Richtung (68) aus einer ersten Tastenposition in einen zweite Tastenposition bewegbar ist;
einem Hebel (70), der um eine Schwenkachse (78) schwenkbar ist,
wobei die zweite Richtung (68) von der ersten Richtung (48) verschieden ist,
wobei der Hebel (70) die Drucktaste (60) und die Wirkeinrichtung (40) derart mechanisch koppelt, dass eine Bewegung der Drucktaste (60) in der zweiten Richtung (68) mit einer Bewegung der Wirkeinrichtung (40) in der ersten Richtung (48) einher geht;
wobei der Hebel (70) im Bereich seiner Schwenkachse (78) keine zur Schwenkachse (78) rotationssymmetrische Fläche aufweist,
**dadurch gekennzeichnet, dass** ein mittlerer Bereich (75) des Hebels (70) durch eine Öffnung (57) in einer Wand (50) zwischen der Drucktaste (60) und der Wirkeinrichtung (40) hindurch ragt,
der mittlere Bereich (75) des Hebels (70) durch die Öffnung (57) in der Wand (50) mechanisch so geführt ist, dass der Hebel (70) um die Schwenkachse (78) schwenkbar, jedoch nicht wesentlich in der ersten Richtung (48) oder in der zweiten Richtung (68) verschiebbar ist.

2. Medizinisches Instrument (10) nach einem der vorangehenden Ansprüche, bei dem die Wand (50) in der Umgebung der Öffnung (57) eine reduzierte Dicke aufweist.

3. Medizinisches Instrument (10) nach einem der vorangehenden Ansprüche, bei dem
der Hebel (70) ein erstes Ende (74) aufweist, das in eine Ausnehmung (47) in der Wirkeinrichtung (40) eingreift,
der Hebel (70) ein zweites Ende (76) aufweist, das in eine Ausnehmung (67) in der Drucktaste (60) eingreift.

4. Medizinisches Instrument (10) nach einem der vorangehenden Ansprüche, ferner mit:
einem O-Ring (80) aus einem elastischen Material,
wobei entweder der O-Ring (80) am zweiten Ende (76) des Hebels (70) anliegt und das erste Ende (74) des Hebels (70) in die Ausnehmung (47) in der Wirkeinrichtung (40) schiebt oder der O-Ring (80) am ersten Ende (74) des Hebels (70) anliegt und das zweite Ende (76) des Hebels (70) in die Ausnehmung (67) in der Drucktaste schiebt.

5. Medizinisches Instrument (10) nach einem der vorangehenden Ansprüche, bei dem
die Wirkeinrichtung ein Arretierungsschieber (40) ist oder mit einem Arretierungsschieber (40) mechanisch gekoppelt ist,
der Arretierungsschieber (40) zum formschlüssigen Halten eines Bestandteils (20) des medizinischen Instruments (10) in einer vorbestimmten Position vorgesehen ist.

6. Medizinisches Instrument (10) nach einem der vorangehenden Ansprüche, bei dem die Wand (50) die Wirkeinrichtung (40) ringförmig umgibt,
ein Bereich (61, 63) der Drucktaste (60) die Wand (50) ringförmig umgibt.

7. Medizinisches Instrument (10) nach einem der vorangehenden Ansprüche, bei dem die Wirkeinrichtung (40) formschlüssig durch die Wand (50) geführt ist,
die Drucktaste (60) formschlüssig durch die Wand (50) geführt ist.

8. Medizinisches Instrument (10) nach einem der vorangehenden Ansprüche, bei dem die Drucktaste (60) ein becherförmiges Bauteil (61) und ein in einen Hohlraum des becherförmigen Bauteils (61) eingesetztes ringförmiges Bauteil (63) umfasst, die Ausnehmung (67) in der Drucktaste (60) in dem ringförmigen Bauteil (63) ausgebildet ist.

9. Medizinisches Instrument (10) nach dem vorangehenden Anspruch in Rückbezug auf Anspruch 5, bei dem das ringförmige Bauteil (63) eine ringförmige an seinem äußeren Umfang umlaufende Nut (64) aufweist, in die der O-Ring (80) eingelegt ist.

10. Medizinisches Instrument (10) nach einem der Ansprüche 8 und 9, bei dem die Ausnehmung in der Drucktaste (60) durch eine fensterförmige Öffnung oder eine Durchgangsbohrung (67) in dem ringförmigen Bauteil (63) gebildet ist.

11. Medizinisches Instrument (10) nach einem der vorangehenden Ansprüche, bei dem Formschluss zwischen dem Hebel (70) und der Drucktaste (60) und Formschluss zwischen dem Hebel (70) und der Öffnung (57) in der Wand (50) ein Abziehen der Drucktaste (60) von dem medizinischen Instrument (10) unterbinden.

12. Medizinisches Instrument (10) nach einem der vorangehenden Ansprüche, bei dem das medizinische Instrument ein Hysteroskop (10) mit einem Endoskop (20) und einem Außenschaft (30) ist,
das Endoskop (20) relativ zum Außenschaft (30) in axialer Richtung verschiebbar ist,
die Wirkeinrichtung (40) eine Arretiereinrichtung ist,
die Wirkeinrichtung (40) vorgesehen und ausgebildet ist, um in der ersten Wirkposition in eine Ausnehmung (24) am Endoskop (20) einzugreifen, um so das Endoskop (20) in einer vorbestimmten Position relativ zum Außenschaft (30) zu arretieren.

13. Verfahren zum Zusammensetzen eines medizinischen Instruments (10) gemäß Anspruch 1 mit folgenden Schritten:
Einsetzen (101) einer Wirkeinrichtung (40) in einen rohrförmigen Bereich (50) des medizinischen Instruments (10);
Einführen (102) des rohrförmigen Bereichs (50) in ein ringförmiges Bauteil (63); Einführen (104) eines Hebels (70) in eine Ausnehmung (67) in dem ringförmigen Bauteil (63), in eine Öffnung (57) im rohrförmigen Bereich (50) und in eine Ausnehmung (47) in der Wirkeinrichtung (40);
Einsetzen (106) des ringförmigen Bauteils (63) in ein becherförmiges Bauteil (61).

## Claims

1. Medical instrument (10), with:
an effecting device (40), which is movable in a first direction (48) from a first effecting position into a second effecting position;
a pushbutton (60), which is movable manually in a second direction (68) from a first button position into a second button position;
a lever (70), which is pivotable about a pivot axis (78),
the second direction (68) being different from the first direction (48),
the lever (70) mechanically coupling the pushbutton (60) and the effecting device (40) in such a way that a movement of the pushbutton (60) in the second direction (68) is accompanied by a movement of the effecting device (40) in the first direction (48) ;
the lever (70) not having an area that is rotationally symmetrical to the pivot axis (78) in the region of its pivot axis (78),
**characterized in that** a middle region (75) of the lever (70) protrudes through an opening (57) in a wall (50) between the pushbutton (60) and the effecting device (40),
the middle region (75) of the lever (70) is mechanically guided through the opening (57) in the wall (50) such that the lever (70) is pivotable about the pivot axis (78), but is not significantly displaceable in the first direction (48) or the second direction (68).

2. Medical instrument (10) according to one of the preceding claims, in which the wall (50) has a reduced thickness in the vicinity of the opening (57).

3. Medical instrument (10) according to one of the preceding claims, in which
the lever (70) has a first end (74), which engages in a clearance (47) in the effecting device (40),
the lever (70) has a second end (76), which engages in a clearance (67) in the pushbutton (60).

4. Medical instrument (10) according to one of the preceding claims, also with:
an 0-ring (80) of an elastic material,
either the 0-ring (80) abutting on the second end (76) of the lever (70) and pushing the first end (74) of the lever (70) into the clearance (47) in the effecting device (40) or the 0-ring (80) abutting on the first end (74) of the lever (70) and pushing the second end (76) of the lever (70) into the clearance (67) in the pushbutton.

5. Medical instrument (10) according to one of the preceding claims, in which
the effecting device is an arresting slide (40) or is mechanically coupled with an arresting slide (40),
the arresting slide (40) is intended for the interlocking retention of a component part (20) of the mechanical instrument (10) in a predetermined position.

6. Medical instrument (10) according to one of the preceding claims, in which
the wall (50) surrounds the effecting device (40) annularly,
a region (61, 63) of the pushbutton (60) surrounds the wall (50) annularly.

7. Medical instrument (10) according to one of the preceding claims, in which
the effecting device (40) is guided through the wall (50) in an interlocking manner,
the pushbutton (60) is guided through the wall (50) in an interlocking manner.

8. Medical instrument (10) according to one of the preceding claims, in which
the pushbutton (60) comprises a cup-shaped component (61) and an annular component (63) inserted into a cavity of the cup-shaped component (61),
the clearance (67) in the pushbutton (60) is formed in the annular component (63).

9. Medical instrument (10) according to the preceding claim with reference back to Claim 5, in which the annular component (63) has a groove (64) that runs around annularly on its outer circumference and in which the 0-ring (80) is placed.

10. Medical instrument (10) according to either of Claims 8 and 9, in which the clearance in the pushbutton (60) is formed by a window-shaped opening or a through-bore (67) in the annular component (63).

11. Medical instrument (10) according to one of the preceding claims, in which
interlocking between the lever (70) and the pushbutton (60) and interlocking between the lever (70) and the opening (57) in the wall (50) prevent the pushbutton (60) from being pulled off from the medical instrument (10).

12. Medical instrument (10) according to one of the preceding claims, in which
the medical instrument is a hysteroscope (10) with an endoscope (20) and an outer shank (30),
the endoscope (20) is displaceable in relation to the outer shank (30) in an axial direction,
the effecting device (40) is an arresting device, the effecting device (40) is intended and designed to engage in a clearance (24) on the endoscope (20) in the first effecting position, in order in this way to arrest the endoscope (20) in a predetermined position in relation to the outer shank (30).

13. Method for assembling a medical instrument (10) according to Claim 1 with the following steps:
inserting (101) an effecting device (40) into a tubular region (50) of the medical instrument (10);
introducing (102) the tubular region (50) into an annular component (63);
introducing (104) a lever (70) into a clearance (67) in the annular component (63), into an opening (57) in the tubular region (50) and into a clearance (47) in the effecting device (40);
inserting (106) the annular component (63) into a cup-shaped component (61).

## Revendications

1. Instrument médical (10), comprenant :
un dispositif d'actionnement (40) qui peut être déplacé dans une première direction (48) d'une première position fonctionnelle dans une deuxième position fonctionnelle ;
un bouton-poussoir (60) qui peut être déplacé manuellement dans une deuxième direction (68) d'une première position de bouton dans une deuxième position de bouton ;
un levier (70) qui peut pivoter autour d'un axe de pivotement (78),
la deuxième direction (68) étant différente de la première direction (48),
le levier (70) accouplant mécaniquement le bouton-poussoir (60) et le dispositif d'actionnement (40) de telle sorte qu'un déplacement du bouton-poussoir (60) dans la deuxième direction (68) s'accompagne d'un déplacement du dispositif d'actionnement (40) dans la première direction (48) ;
le levier (70), dans la région de son axe de pivotement (78), ne présentant pas une surface à symétrie de révolution par rapport à l'axe de pivotement (78),
**caractérisé en ce**
**qu'**une région centrale (75) du levier (70) pénètre à travers une ouverture (57) dans une paroi (50) entre le bouton-poussoir (60) et le dispositif d'actionnement (40), et
la région centrale (75) du levier (70) est guidée mécaniquement à travers l'ouverture (57) dans la paroi (50) de telle sorte que le levier (70) puisse pivoter autour de l'axe de pivotement (78) mais ne puisse toutefois pas être déplacé sensiblement dans la première direction (48) ou dans la deuxième direction (68).

2. Instrument médical (10) selon l'une quelconque des revendications précédentes, dans lequel la paroi (50) présente une épaisseur réduite dans l'environnement de l'ouverture (57).

3. Instrument médical (10) selon l'une quelconque des revendications précédentes, dans lequel le levier (70) présente une première extrémité (74) qui vient en prise dans un évidement (47) dans le dispositif d'actionnement (40),
le levier (70) présente une deuxième extrémité (76) qui vient en prise dans un évidement (67) dans le bouton-poussoir (60).

4. Instrument médical (10) selon l'une quelconque des revendications précédentes, comprenant en outre :
un joint torique (80) en matériau élastique,
soit le joint torique (80) s'appliquant contre la deuxième extrémité (76) du levier (70) et poussant la première extrémité (74) du levier (70) dans l'évidement (47) dans le dispositif d'actionnement (40) soit le joint torique (80) s'appliquant contre la première extrémité (74) du levier (70) et poussant la deuxième extrémité (76) du levier (70) dans l'évidement (67) dans le bouton-poussoir.

5. Instrument médical (10) selon l'une quelconque des revendications précédentes, dans lequel le dispositif d'actionnement est un coulisseau de blocage (40) ou est accouplé mécaniquement à un coulisseau de blocage (40),
le coulisseau de blocage (40) est prévu dans une position prédéterminée pour retenir par engagement par correspondance de formes une pièce (20) de l'instrument médical (10).

6. Instrument médical (10) selon l'une quelconque des revendications précédentes, dans lequel la paroi (50) entoure sous forme annulaire le dispositif d'actionnement (40),
une région (61, 63) du bouton-poussoir (60) entoure sous forme annulaire la paroi (50).

7. Instrument médical (10) selon l'une quelconque des revendications précédentes, dans lequel le dispositif d'actionnement (40) est guidé par engagement par correspondance de formes à travers la paroi (50) et
le bouton-poussoir (60) est guidé par engagement par correspondance de formes à travers la paroi (50).

8. Instrument médical (10) selon l'une quelconque des revendications précédentes, dans lequel le bouton-poussoir (60) comprend un composant en forme de godet (61) et un composant de forme annulaire (63) inséré dans une cavité du composant en forme de godet (61),
l'évidement (67) est réalisé dans le bouton-poussoir (60) dans le composant de forme annulaire (63).

9. Instrument médical (10) selon la revendication précédente lorsqu'elle se rapporte à la revendication 5, dans lequel le composant de forme annulaire (63) présente une rainure périphérique de forme annulaire (64) s'étendant sur sa périphérie extérieure, dans laquelle est inséré le joint torique (80).

10. Instrument médical (10) selon l'une quelconque des revendications 8 et 9, dans lequel l'évidement dans le bouton-poussoir (60) est formé par une ouverture en forme de fenêtre ou par un alésage traversant (67) dans le composant de forme annulaire (63).

11. Instrument médical (10) selon l'une quelconque des revendications précédentes, dans lequel l'engagement par correspondance de formes entre le levier (70) et le bouton-poussoir (60) et l'engagement par correspondance de formes entre le levier (70) et l'ouverture (57) dans la paroi (50) empêchent un retrait du bouton-poussoir (60) de l'instrument médical (10).

12. Instrument médical (10) selon l'une quelconque des revendications précédentes, dans lequel l'instrument médical est un hystéroscope (10) avec un endoscope (20) et une tige extérieure (30), l'endoscope (20) peut être déplacé dans la direction axiale par rapport à la tige extérieure (30),
le dispositif d'actionnement (40) est un dispositif de blocage, et
le dispositif d'actionnement (40) est prévu et réalisé pour venir en prise dans la première position fonctionnelle dans un évidement (24) sur l'endoscope (20) afin de bloquer l'endoscope (20) dans une position prédéfinie par rapport à la tige extérieure (30).

13. Procédé d'assemblage d'un instrument médical (10) selon la revendication 1, comprenant les étapes suivantes :
insertion (101) d'un dispositif d'actionnement (40) dans une région de forme tubulaire (50) de l'instrument médical (10) ;
introduction (102) de la région de forme tubulaire (50) dans un composant de forme annulaire (63) ;
introduction (104) d'un levier (70) dans un évidement (67) dans le composant de forme annulaire (63), dans une ouverture (57) dans la région de forme tubulaire (50) et dans un évidement (47) dans le dispositif d'actionnement (40) ; et
insertion (106) du composant de forme annulaire (63) dans un composant en forme de godet (61).
